# EUROPEAN PATENT SPECIFICATION

(11) **EP 0 630 376 B1**
(45) Date of publication and mention of the grant of the patent: **02.06.1999**
(21) Application number: 93905561.2
(22) Date of filing: 10.03.1993
(51) Int. Cl.: C07D 487/04, C07D 498/04, C07D 513/04, C07D 471/04, C07D 519/00, A61K 31/445, A61K 31/535

(54) **CONDENSED INDOLE DERIVATIVES AS 5-HT4-RECEPTOR ANTAGONISTS**
KONDENSIERTE INDOL-DERIVATE ALS 5-HT4-REZEPTOR-ANTAGONISTEN
DERIVES CONDENSES D'INDOLES EN TANT QU'ANTAGONISTES DU RECEPTEUR 5-HT4

(30) Priority: 12.03.1992 GB 9205428; 05.09.1992 GB 9218846; 29.12.1992 GB 9227045
(43) Date of publication of application: 28.12.1994
(60) Divisional application: 98114130.2
(73) Proprietor: SMITHKLINE BEECHAM PLC, Brentford, Middlesex TW8 9EP (GB)
(72) Inventor: GASTER, Laramie Mary, SmithKline Beecham Parm., Harlow, Essex CM 19 5AD (GB); WYMAN, Paul Adrian, SmithKline Beecham Parm., Harlow, Essex CM19 5AD (GB)
(74) Representative: Tocher, Pauline
(86) International application number: GB9300506
(87) International publication number: WO9318036

(56) References cited:
- EP-A- 0 429 984
- EP-A- 0 485 962
- EP-A- 0 501 322
- GB-A- 1 566 307

## Description

This invention relates to novel compounds having pharmacological activity, to a process for their preparation and to their use as pharmaceuticals.

EP-A-429984 (Nisshin Flour Milling Co., Ltd.) describes indole derivatives having 5-HT₃ receptor antagonist activity.

European Journal of Pharmacology 146 (1988), 187-188, and Naunyn-Schmiedeberg's Arch. Pharmacol. (1989) 340:403-410, describe a non classical 5-hydroxytryptamine receptor, now designated the 5-HT₄ receptor, and that ICS 205-930, which is also a 5-HT₃ receptor antagonist, acts as an antagonist at this receptor.

WO 91/16045 (SmithKline and French Laboratories Limited) describes the use of cardiac 5-HT₄ receptor antagonists in the treatment of atrial arrhythmias and stroke.

EP-A-501322 (Glaxo Group Limited) describes indole derivatives having 5-HT₄ antagonist activity.

A class of novel, structurally distinct compounds has now been discovered, which compounds are indole derivatives 1,2-disubstituted by alkyleneoxy, with an azacyclic, fused azabicyclic or aminoalkyl moiety. These compounds have 5-HT₄ receptor antagonist activity.

Accordingly, the present invention provides a compound of formula (I), or a pharmaceutically acceptable salt thereof: 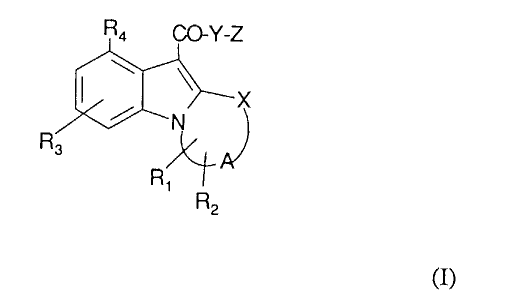 wherein
X is O, S, SO, SO₂, CH₂, CH or NR wherein R is hydrogen, C₁₋₆ alkyl or C₃₋₆ cycloalkyl;
A is a saturated or unsaturated polymethylene chain of 2 - 4 carbon atoms;
R₁ and R₂ are hydrogen, C₁₋₆ alkyl or C₃₋₆ cycloalkyl;
R₃ is hydrogen, halo, C₁₋₆ alkyl, C₃₋₆ cycloalkyl, amino, nitro or C₁₋₆ alkoxy;
R₄ is hydrogen, halo, C₁₋₆ alkyl, C₃₋₆ cycloalkyl or C₁₋₆ alkoxy;
Y is O or NH;
Z is of sub-formula (a), (b) or (c): 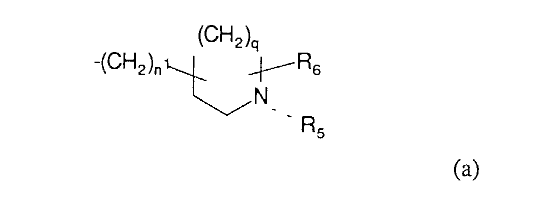 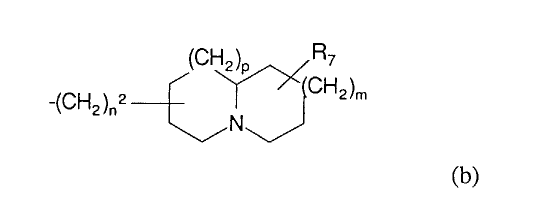 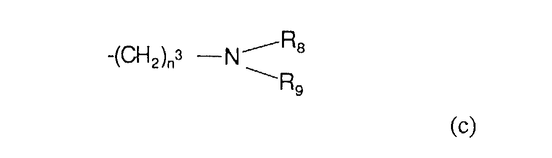 wherein
n¹ is is 1, 2, 3 or 4; n² is 0, 1, 2, 3 or 4; n³ is 2, 3, 4 or 5;
q is 0, 1, 2 or 3; p is 0, 1 or 2; m is 0, 1 or 2;
R₅ is hydrogen, C₁₋₁₂ alkyl, aralkyl or R₅ is (CH₂)_{z}-R₁₀ wherein z is 2 or 3 and
   R₁₀ is selected from cyano, hydroxyl, C₁₋₆ alkoxy, phenoxy, C(O)C₁₋₆ alkyl, COC₆H₅, -CONR₁₁R₁₂, NR₁₁COR₁₂, SO₂NR₁₁R₁₂ or NR₁₁SO₂R₁₂
   wherein R₁₁ and R₁₂ are hydrogen, C₁₋₆ alkyl or C₃₋₆ cycloalkyl;
R₆, R₇ and R₈ are independently hydrogen, C₁₋₆ alkyl or C₃₋₆ cycloalkyl ;
R₉ is hydrogen, C₁₋₆ alkyl or C₃₋₆ cycloalkyl; and
the dotted line in sub-formula (a) indicates the R₅ group is absent when the -(CH₂)ₙ1- group is directly attached to the nitrogen of the azacycle; or a compound of formula (I) wherein the CO-Y linkage is replaced by a heterocyclic bioisostere of formula (d): 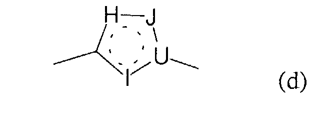 wherein
the dotted circle represents one or two double bonds in any position in the 5-membered ring; H, J and I independently represent oxygen, sulphur, nitrogen or carbon, provided that at least one of H, J and I is other than carbon; U represents nitrogen or carbon;
other than the compound of formula (I) in which X is O, A is -(CH₂)₃-, R₁ R₂ R₃ and R₄ are hydrogen, Y is NH, Z is of sub-formula (a) in which n¹ is 1, q is 3, R₅ is n-butyl and R₆ is hydrogen;
having 5-HT₄ receptor antagonist activity.

Examples of alkyl or alkyl containing groups include C₁, C₂, C₃, C₄, C₅, C₆, C₇, C₈, C₉, C₁₀, C₁₁ or C₁₂ branched, straight chained or cyclic alkyl, as appropriate. C₁₋₄ alkyl groups include methyl, ethyl, *n-* and iso-propyl, *n-, iso-, sec-* and *tert*-butyl. Cyclic alkyl includes cyclopropyl, cyclobutyl, cyclopentyl, cyclohexyl, cycloheptyl and cyclooctyl.

Aryl includes phenyl and naphthyl optionally substituted by one or more substituents selected from halo, C₁₋₆ alkyl and C₁₋₆ alkoxy.

Halo includes fluoro, chloro, bromo and iodo.

Suitable examples of (d) are as described for X, Y and Z in EP-A-328200 (Merck Sharp & Dohme Ltd.), such as an oxadiazole moiety.

X is often O.

Values for A include -CH₂-(CH₂)ᵣ-CH₂- wherein r is 0, 1 or 2; -CH₂-CH=CH-; -C(CH₃)=CH- or when X is CH or N, A may be -(CH₂)₂-CH= or -CH=CH-CH=. Other examples of A are as described in the examples hereinafter.

R₁ and R₂ are often hydrogen or R₁ and R₂ are gem-dimethyl.

r is often 1.

R₃ is preferably hydrogen.

R₄ is preferably hydrogen or halo, such as fluoro.

Y is preferably O or NH.

When Z is of sub-formula (a), n¹ is preferably 2, 3 or 4 when the azacycle is attached at the nitrogen atom and n¹ is preferably 1 when the azacycle is attached at a carbon atom, such as the 4-position when q is 2.

When Z is of sub-formula (b), n² is preferably such that the number of carbon atoms between the ester or amide linkage is from 2 to 4 carbon atoms.

Suitable values for p and m include p = m = 1; p = 0, m = 1, p = 1, m = 2, p = 2, m = 1.

When Z is of sub-formula (c), n³ is preferably 2, 3 or 4.

R₉ and R₁₀ are preferably both alkyl, especially one of R₉ and R₁₀ is C₄ or larger alkyl.

Specific values of Z of particular interest are as follows:

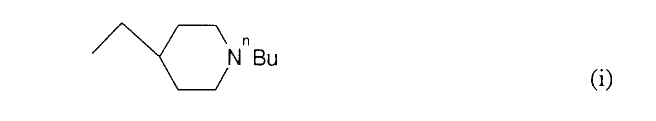

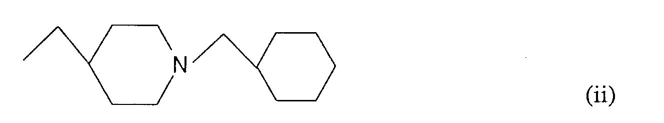

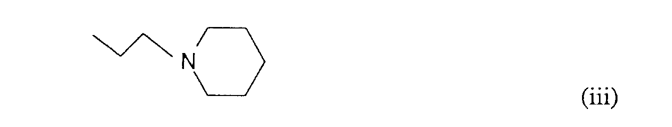

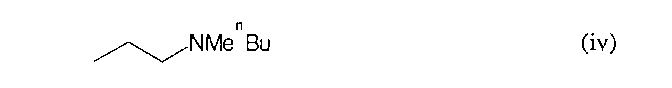

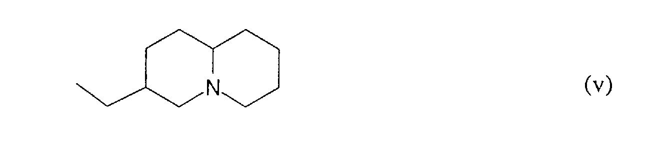

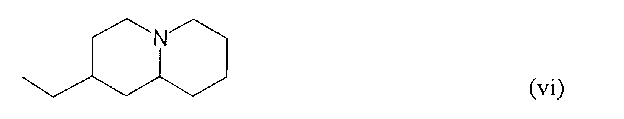

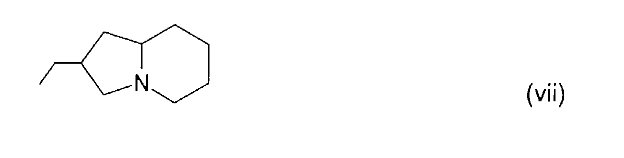

The invention also provides novel compounds within formula (I) with side chains (i), (ii), (iii), (iv), (v), (vi) or (vii). In a further aspect, the piperidine ring in (i), (ii) or (iii) may be replaced by pyrrolidinyl or azetidinyl, and/or the N-substituent in (i) or (ii) may be replaced by C₃ or larger alkyl or optionally substituted benzyl.

In an alternative aspect, the N-substituent in formula (i) or (ii) may be replaced by (CH₂)ₙR⁴ as defined in formula (I) and in relation to the specific examples of EP-A-501322.

The pharmaceutically acceptable salts of the compounds of the formula (I) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

Examples of pharmaceutically acceptable salts include quaternary derivatives of the compounds of formula (I) such as the compounds quaternised by compounds Rₓ-T wherein Rₓ is C₁₋₆ alkyl, phenyl-C₁₋₆ alkyl or C₅₋₇ cycloalkyl, and T is a radical corresponding to an anion of an acid. Suitable examples of Rₓ include methyl, ethyl and *n-* and *iso*-propyl; and benzyl and phenethyl. Suitable examples of T include halide such as chloride, bromide and iodide.

Examples of pharmaceutically acceptable salts also include internal salts such as N-oxides.

The compounds of the formula (I), their pharmaceutically acceptable salts, (including quaternary derivatives and N-oxides) may also form pharmaceutically acceptable solvates, such as hydrates, which are included wherever a compound of formula (I) or a salt thereof is herein referred to.

It will also be realised that the (CH₂)ₙ2 moiety in compounds of formula (I) wherein Z is (b), may adopt an α or β or configuration with respect to the fused azabicyclic moiety.

The compounds of formula (I) may be prepared by conventional coupling of the indole moiety with Z. Suitable methods are as described in GB 2125398A (Sandoz Limited), GB 1593146A and EP-A-36269 (Beecham Group p.l.c.), EP-A-429984 (Nisshin Flour Milling Co.) and EP-A-328200 (Merck Sharp & Dohme Limited). Reference is also made to EP-A-501322 (Glaxo Group Limited). It will be appreciated that the (CH₂)ᵣ-O containing ring or R₃/R₄ introduction/modification may be carried out before or after coupling.

Aza(bi)cyclic side chain intermediates are known compounds or may be prepared according to the methods described in PCT/GB92/01519 and /01612 (SmithKline Beecham p.l.c.).

The compounds of the present invention are 5-HT₄ receptor antagonists and it is thus believed may generally be used in the treatment or prophylaxis of gastrointestinal disorders, cardiovascular disorders and CNS disorders.

They are of potential interest in the treatment of irritable bowel syndrome (IBS), in particular the diarrhoea aspects of IBS, i.e., these compounds block the ability of 5-HT to stimulate gut motility via activation of enteric neurones. In animal models of IBS, this can be conveniently measured as a reduction of the rate of defaecation. They are also of potential use in the treatment of urinary incontinence which is often associated with IBS.

They may also be of potential use in other gastrointestinal disorders, such as those associated with upper gut motility, and as antiemetics. In particular, they are of potential use in the treatment of the nausea and gastric symptoms of gastro-oesophageal reflux disease and dyspepsia. Antiemetic activity is determined in known animal models of cytotoxic-agent/radiation induced emesis.

Specific cardiac 5-HT₄ receptor antagonists which prevent atrial fibrillation and other atrial arrhythmias associated with 5-HT, would also be expected to reduce occurrence of stroke (see A.J. Kaumann 1990, Naumyn-Schmiedeberg's Arch. Pharmacol. 342, 619-622, for appropriate animal test method).

It is believed that platelet-derived 5-HT induces atrial arrhythmias which encourage atrial fibrillation and atrial disorders are associated with symptomatic cerebral and sytemic embolism. Cerebral embolism is the most common cause of ischaemic stroke and the heart the most common source of embolic material. Of particular concern is the frequency of embolism associated with atrial fibrillation.

Anxiolytic activity is likely to be effected via the hippocampus (Dumuis *et al* 1988, Mol Pharmacol., 34, 880-887). Activity may be demonstrated in standard animal models, the social interaction test and the X-maze test.

Migraine sufferers often undergo situations of anxiety and emotional stress that precede the appearance of headache (Sachs, 1985, Migraine, Pan Books, London). It has also been observed that during and within 48 hours of a migraine attack, cyclic AMP levels are considerably increased in the cerebrospinal fluid (Welch *et al.,* 1976, Headache 16, 160-167). It is believed that a migraine, including the prodomal phase and the associated increased levels of cyclic AMP are related to stimulation of 5-HT₄ receptors, and hence that administration of a 5-HT₄ antagonist is of potential benefit in relieving a migraine attack.

The invention also provides a pharmaceutical composition comprising a compound of formula (I), or a pharmaceutically acceptable salt thereof, and a pharmaceutically acceptable carrier.

Such compositions are prepared by admixture and are usually adapted for enteral such as oral, nasal or rectal, or parenteral administration, and as such may be in the form of tablets, capsules, oral liquid preparations, powders, granules, lozenges, reconstitutable powders, nasal sprays, suppositories, injectable and infusable solutions or suspensions. Sublingual or transdermal administration is also envisaged. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The invention further provides a method of treatment of irritable bowel syndrome, gastro-oesophagal reflux disease, dyspepsia, atrial arrhythmias and stroke, anxiety and/or migraine in mammals, such as humans, which comprises the administration of an effective amount of a compound of the formula (I) or a pharmaceutically acceptable salt thereof. In particular, the method comprises treatment of IBS or atrial arrhythmias and stroke.

An amount effective to treat the disorders hereinbefore described depends on the relative efficacies of the compounds of the invention, the nature and severity of the disorder being treated and the weight of the mammal. However, a unit dose for a 70 kg adult will normally contain 0.05 to 1000 mg for example 0.5 to 500 mg, of the compound of the invention. Unit doses may be administered once or more than once a day, for example, 2, 3 or 4 times a day, more usually 1 to 3 times a day, that is in the range of approximately 0.0001 to 50 mg/kg/day, more usually 0.0002 to 25 mg/kg/day.

No adverse toxicological effects are indicated within the aforementioned dosage ranges.

The invention also provides a compound of formula (I) or a pharmaceutically acceptable salt thereof for use as an active therapeutic substance, in particular for use as a 5-HT₄ receptor antagonist in the treatment of the disorders hereinbefore described.

The invention also provides the use of a compound of formula (I) in the manufacture of a medicament for use as a 5-HT₄ receptor antagonist in the treatment of the disorders hereinbefore described.

The following Examples illustrate the preparation of compounds of formula (I); the following Descriptions illustrate the preparation of intermediates. The compound described in Example 3 (E3) is the subject of the divisional application of the present application.

| Examples | | | | | | | | |
|---|---|---|---|---|---|---|---|---|
| | **R**_{**1**} | **R**_{**2**} | **r** | **R**_{**3**} | **R**_{**4**} | **X** | **Y** | **Z** |
| **E1** | H | H | 1 | H | H | O | O | (i) |
| **E2** | H | H | 1 | H | H | O | O | (vi) |
| **E3** | H | H | 1 | H | H | O | NH | (i) |
| **E4** | H | H | 1 | H | H | O | O | (iii) |
| **E5** | H | H | 1 | H | H | O | NH | (iii) |
| **E6** | H | H | O | H | H | O | O | (i) |
| **E7** | 3-(CH₃)₂ | | 1 | H | H | O | O | (i) |
| **E8** | H | H | 1 | H | H | S | O | (i) |
| **E9** | H | H | 2 | H | H | O | O | (i) |
| **E10** | H | H | 1 | H | H | CH₂ | O | (i) |
| **E11** | H | H | O | H | H | CH₂ | O | (i) |
| **E12** | H | H | 2 | H | H | CH₂ | O | (i) |
| **E13** | H | H | O | H | H | CH₂ | NH | (i) |
| **E14** | H | H | O | H | H | O | NH | (i) |
| **E15** | H | H | 1 | H | H | O | O | Bzppm |
| **E16** | H | H | 1 | H | H | SO | O | (i) |
| **E17** | -- | Δ | - | H | H | CH | O | (i) |
| **E18** | -- | τ | - | H | H | CH | O | (i) |
| **E19** | H | H | 1 | H H | H | S | NH | (i) |
| **E20** | H | H | 1 | H | H | O | NH | Bzppm |
| **E21** | H | H | 1 | H | H | O | NH | ppm |
| **E22** | H | H | 1 | H | H | O | NH | ⁿC₆H₁₃ppm |
| **E23** | H | H | 1 | H | H | O | NH | (ii) |
| **E24** | H | H | 1 | H | H | O | NH | Etppm |
| **E25** | H | H | 1 | H | H | O | NH | MeSO₂aEtppm |
| **E26** | H | H | 1 | H | H | O | NH | (vi) |
| **E27** | H | H | 1 | 8-F | H | O | O | (i) |
| **E28** | H | H | 1 | 8-F | H | O | NH | (i) |
| **E29** | H | H | 1 | H | H | NMe | O | (i) |
| **E30** | - | π | -- | H | H | S | O | (i) |
| **E31** | H | H | 0 | H | H | S | O | (i) |
| **E32** | -- | ϑ | - | H | H | S | O | (i) |
| **E33** | -- | Λ | - | H | H | N | O | (i) |
| **E34** | H | H | 0 | H | H | S | NH | (i) |
| **E35** | -- | ϑ | - | H | H | S | NH | (i) |
| **E36** | H | H | 1 | H | H | NH | O | (i) |
| **E37** | H | H | 0 | H | H | O | O | (vi) |
| **E38** | H | H | 2 | H | H | O | NH | (i) |
| **E39** | H | τ | - | H | H | N | O | (i) |
| **E40** | H | H | 0 | H | H | S | O | (vi) |
| **E41** | H | H | 0 | H | H | S | NH | (vi) |
| **E42** | -- | ϑ | -- | H | H | S | O | (vi) |
| **E43** | -- | ϑ | -- | H | H | S | NH | (vi) |
| **E44** | H | H | 1 | H | H | S | O | (vi) |
| **E45** | -- | Γ | -- | H | H | NH | NH | (i) |
| **E46** | H | H | 1 | H | H | N | NH | (i) |
| Δ - AR₁R₂ is -(CH₂)₂-CH= Bz - benzyl Γ - AR₁R₂ is -CH=CH-CH= ppm - 4-piperidylmethyl π - AR₁R₂ is -C(CH₃)=CH- aEt - aminoethyl ϑ - AR₁R₂ is -CH=CH- Λ - AR₁R₂ is -C(CH₃)=CH-C(CH₃)= | | | | | | | | |

### Example 1

### (1-ⁿButyl-4-piperidyl)methyl-3,4-dihydro-2H-[1,3]oxazino[3,2-a]indole-10-carboxylate (E1)

a) A suspension of indole-3-carboxylic acid (500mg, 0.003 mole) in dichloromethane (50 ml) was treated with oxalyl chloride (0.635g, 0.005 mole) and two drops of dimethylformamide. The mixture was stirred at room temperature for one and a half hours then the solvent was removed *in vacuo* to leave the acid chloride.
   A solution of 1-butyl-4-piperidinemethanol, D6, (513 mg, 0.003 mole) in dry THF (10 ml) under an atmosphere of nitrogen, was cooled in an ice bath. n-Butyllithium (1.88 ml of 1.6M solution in hexane, 0.003 mole) was added dropwise and the resulting solution stirred at 0°C for 15 minutes.
   The acid chloride was dissolved in dry THF (20 ml) and the solution added dropwise to the solution of the lithium alkoxide at 0°C.
   The reaction mixture was allowed to warm to room temperature and was stirred for 3 hours. The solvent was removed *in vacuo* and the residue partitioned between chloroform and water. The chloroform was separated, washed several times with water, dried and concentrated to give (1-butyl-4-piperidyl)methyl-1H-indole-3-carboxylate as a pale brown gum.
   ¹H NMR (250 MHz) CDCl₃;
   δ:9.90 (br s, 1H), 8.10-8.18 (m, 1H), 7.78 (d, 1H), 7.37-7.46 (m, 1H), 7.16-7.28 (m, 2H), 4.19 (d, 2H), 3.05-3.15 (br d, 2H), 2.40-2.49 (m, 2H), 0.90 (t, 3H), 1.20-2.18 (m, 11H).
b) A suspension of N-chlorosuccinimide (57mg, 0.48 mmole) in chloroform (2ml) was treated with a solution of (1-ⁿbutyl-4-piperidyl)methyl indole-3-carboxylate (100mg, 0.32 mmole) in chloroform (2ml) and the mixture stirred at room temperature for 2h. The pale yellow solution was treated with 3-bromo-1-propanol (0.03ml, 0.32 mmole), stirred at room temperature for 16 h; then basified with 10% Na₂CO₃ solution and extracted with chloroform. The extract was dried and concentrated to leave a yellow gum, which was dissolved in acetone (6ml), treated with anhydrous potassium carbonate (130mg, 0.94 mmole) and stirred at room temperature for 18 h. The mixture was treated with 10% Na₂CO₃ solution and extracted with ethyl acetate. The extract was dried and concentrated to leave a brown oil, which was chromatographed, first on silica gel eluting with chloroform/methanol (97:3), then on basic alumina eluting with ethyl acetate to give a colourless oil. This was crystallised from ether/pentane to afford the title compound (E1) as a white solid (11mg) mp 117-119°C.
   ¹H NMR (CDCl₃)
   δ: 7.97 (d,1H), 7.10-7.30 (m,3H), 4.55 (t,2H), 4.20 (d,2H), 4.11 (t,2H), 2.90-3.03 (m,2H), 2.25-2.40 (m,4H), 1.75-2.00 (m,5H), 1.22-1.55 (m,6H), 0.91 (t,3H)
   MS (E1) M⁺ 370

### Example 2

### eq-Quinolizidin-2-ylmethyl-3,4-dihydro-2H-[1,3]oxazino[3,2-a]indol-10-carboxylate (E2)

a) *eq*-2-Hydroxymethylquinolizidine (N.J. Leonard et al., J. Org. Chem., 1957, 22, 1445) was reacted with indole-3-carboxylic acid chloride using the method described in Example 1a to afford eq-quinolizidin-2-ylmethyl 1-H-indole-3-carboxylate mp 154-157°C.
   ¹H NMR (CDCl₃)
   δ : 9.40 (br.s,1H), 8.10-8.20 (m,1H), 7.87 (d,1H), 7.35-7.45 (m,1H), 7.20-7.30 (m,2H), 4.20 (d,2H), 2.80-2.97 (m,2H), 1.43-2.20 (m,11H), 1.10-1.40 (m,3H).
b) *eq*-Quinolizidin-2-ylmethyl 1 H-indole-3-carboxylate was treated initially with N-chlorosuccinimide (1.5 equivalents) for 2h, then with 3-bromo-1-propanol (2 equivalents) for 16h, followed by anhydrous potassium carbonate in acetone, using the method described in Example 1b. The crude product was purified using the same chromatography conditions as in Example 1b to afford the title compound as a colourless oil (51%). This was converted to its hydrochloride salt and crystallised from acetone mp 164-167°C.
   ¹H NMR (HCI salt) (d⁶DMSO)
   δ: 10.35 (br.s,1H), 7.85 (d,1H), 7.32 (d,1H), 7.07-7.20 (m,2H), 4.54 (t,2H), 4.13 (t,2H), 4.05 (d,2H), 3.25-3.43 (m,2H), 2.74-3.15 (m,3H), 2.20-2.33 (m,2H), 2.00-2.15 (m,1H), 1.35-1.95 (m,10H).

### Example 3

### N-[(1-ⁿButyl-4-piperidyl)methyl]-3,4-dihydro-2H-[1,3]oxazino[3,2-a]indole-10-carboxamide (E3)

**Method 1:-** A stirred solution of N-chlorosuccinimide (57 mg, 0.48 mmole) in chloroform (3 ml) was treated with a solution of N-[(1-ⁿbutyl-4-piperidyl)methyl] indole-3-carboxamide, D1, (100 mg, 0.32 mmole) in chloroform (8 ml) and kept at room temperature for 2h, then treated with 3-bromo-1-propanol (0.03 ml, 0.32 mmole). After stirring for 16h, more 3-bromo-1-propanol (0.03 ml, 0.32 mmole) was added. The mixture was stirred at room temperature for a further 3h, then treated with excess 10% Na₂CO₃ solution and extracted with chloroform. The extract was dried (Na₂SO₄) and concentrated *in vacuo* to leave a yellow oil, which was dissolved in acetone (10 ml), treated with anhydrous potassium carbonate (130 mg, 0.96 mmole) and stirred at room temperature for 16h. The mixture was concentrated *in vacuo,* the residue treated with 10% Na₂CO₃ solution (10 ml) and extracted with chloroform. The extract was dried (Na₂SO₄) and concentrated *in vacuo* to leave a yellow oil, which was chromatographed, initially on silica gel eluting with chloroform/methanol (19:1), then on basic alumina eluting with ethyl acetate. The colourless oil obtained crystallised from ether to afford the title compound (E3) as a white solid (20 mg, 17%) mp 110-113°C.
¹H NMR (CDCl₃)
δ: 8.34 (d,1H), 7.05-7.30 (m,3H), 6.55 (t,1H), 4.53 (t,2H), 4.10 (t,2H), 3.33 (t,2H), 2.90-3.05 (m,2H), 2.25-2.45 (m,4H), 1.90-2.25 (m,2H), 1.20-1.85 (m,9H), 0.92 (t,3H).
MS (Cl) MH⁺ 370.

**Method 2:-** A stirred suspension of N-[(1-ⁿbutyl-4-piperidyl)methyl] indole-3-carboxamide (D1, 120g, 0.38 mole) in chloroform (2 L) under nitrogen at room temperature was treated with freshly distilled 3-bromo-1-propanol (69 ml, 0.77 mole) followed by the portionwise addition of dry N-chlorosuccinimide (55g, 0.42 mole) over 5 minutes. The resulting yellow solution was stirred for 2.5h, then treated with 1M HCI in ether (15 ml, 0.015 mole). A moderate exotherm occurred and the reaction colour changed to orange. After a further 2h the mixture was treated with 10% Na₂CO₃ solution (700 ml) and the chloroform layer separated, dried (Na₂SO₄) and concentrated *in vacuo* to leave a thick red oil. This was treated with acetone (1.5 L) and anhydrous potassium carbonate (130g, 0.95 mole), then stirred at room temperature for 18h. The reaction mixture was concentrated *in vacuo* and the residue treated with water (1 L) and extracted with ethyl acetate (1 L). On standing a solid began crystallising from the ethyl acetate extract. After 2h at 8°C this was filtered off and dried to afford 51.7g of the title compound (E3) as a beige solid. The mother liquors were extracted with 1M HCI acid (800 ml), the acid extract then basified with K₂CO₃ and extracted with chloroform (2 x 700 ml). The combined chloroform extracts were dried (Na₂SO₄), concentrated *in vacuo* and the residue chromatographed on silica gel eluting with chloroform/methanol (96:4). A yellow oil was obtained which upon trituration with ether gave a further 21.3g of title compound (E3) as a white solid. Conversion to the hydrochloride salt and recrystallisation from ethanoV60-80 petrol gave a white solid mp 254-256 °C dec.
HCI salt - ¹H NMR (D₂O)
δ: 7.90 (d,1H), 6.88-7.20 (m,3H), 4.35 (br t,2H), 3.70 (br t,2H), 3.40 (br d,2H), 3.20 (br d,2H), 2.9 (br t,2H), 2.65(br t,2H), 2.12 (br t,2H), 1.20-1.90 (m,9H), 0.87 (t,3H).

Elemental analysis obtained was as follows:

| | Theory | Found | |
|---|---|---|---|
| Carbon | 65.09 | 64.76, | 64.75 |
| Hydrogen | 7.95 | 7.73, | 7.77 |
| Nitrogen | 10.35 | 10.35, | 10.36 |

### Example 4

### 2-(1-Piperidyl)ethyl 3,4-dihydro-2H-[1,3]oxazino[3,2-a]indole-10-carboxylate (E4)

a) 1-Piperidineethanol was reacted with 1H-indole-3-carboxylic acid chloride using the method described in Example 1a to afford 2-(1-piperidyl)ethyl 1 H-indole-3-carboxylate.
   ¹H NMR (CDCl₃)
   δ: 9.6 (br,s, 1H), 8.03-8.12 (m, 1H), 7.73 (d, 1H), 7.30-7.40 (m,1H), 7.13-7.25 (m, 2H), 4.48 (t, 2H), 2.82 (t, 2H), 2.50-2.65 (m, 4H), 1.35-1.70 (m, 6H).
b) 2-(1-Piperidyl)ethyl 1H-indole-3-carboxylate was treated initially with N-chlorosuccinimide (1.5 equivalents) for 2h, then with 3-bromo-1-propanol (3 equivalents) for 21h, followed by anhydrous potassium carbonate in acetone, using the method described in Example 1 b. The crude product was purified using the same chromatography conditions as in Example 1b to afford the title compound (E4) as a pale yellow oil (15%). This was converted to its oxalate salt and crystallised from acetone mp 174-177°C.
   Free base: ¹H NMR (CDCl₃)
   δ: 8.02 (d, 1H), 7.07-7.30 (m,3H), 4.40-4.55 (m, 4H), 4.08 (t, 2H), 2.78 (t, 2H), 2.45-2.65 (m, 4H), 2.25-2.38 (m, 2H), 1.54-1.66 (m, 4H), 1.35-1.50 (m, 2H).
   MS (Cl) MH⁺ 329.

### Example 5

### N-[2-(1-Piperidyl)ethyl] 3,4-dihydro-2H-[1,3]oxazinop,2-alindole-10-carboxamide (E5)

N-[2-(1-Piperidyl)ethyl] 1 H-indole-3-carboxamide (D2) was treated initially with N-chlorosuccinimide, then with 3-bromo-1-propanol, then with potassium carbonate in acetone following the method described in Example 3. The crude product was chromatographed on silica gel eluting with chloroform/methanol (19:1) to give a pale yellow oil, which crystallised from ether to afford the title compound (E5) as a white solid (29%) mp 124-127°C.
¹H NMR (CDCl₃)
δ: 8.33 (d, 1H), 7.06-7.28 (m, 3H), 7.02 (br.t, NH), 4.51 (t, 2H), 4.08 (t, 2H), 3.50-3.60 (m, 2H), 2.54 (t, 2H), 2.30-2.60 (m, 6H), 1.40-1.65 (m, 6H).
MS (Cl) MH⁺ 328.

### Example 6

### (1-ⁿButyl-4-piperidyl)methyl-2,3-dihydrooxazolo[3,2-a]indole-9-carboxylate (E6)

(1-ⁿButyl-4-piperidyl)methyl 1 H-indole-3-carboxylate (E1a) was treated initially with N-chlorosuccinimide (1.5 equivalents) for 4h, then with 2-bromoethanol (2 equivalents) for 18h, followed by anhydrous potassium carbonate in acetone (18h), using the method described in Example 1b. The crude product was purified using the same chromatography conditions as in Example 1b to give a colourless oil (26%), which crystallised from ether to afford the title compound (E6) as a white solid mp 128-130°C.
¹H NMR (CDCl₃)
δ: 7.95-8.02 (m, 1H), 7.07-7.27 (m, 3H), 5.18-5.27 (m, 2H), 4.24-4.33 (m, 2H), 4.19 (d, 2H), 2.92-3.04 (m, 2H), 2.27-2.38 (m, 2H), 1.75-2.05 (m, 5H), 1.25-1.66 (m, 6H), 0.91 (t, 3H).
MS (El) M⁺ 356.

### Example 7

### (1-ⁿButyl-4-piperidyl)methyl-3,3-dimethyl-3,4-dihydro-2H-[1,3]oxazino[3,2-a]indole-10-carboxylate (E7)

(1-ⁿButyl-4-piperidyl)methyl 1H-indole-3-carboxylate (E1a) was treated initially with N-chlorosuccinimide (1.5 equivalents) for 2h, then with 3-bromo-2,2-dimethyl-1-propanol (2 equivalents) for 20h, followed by anhydrous potassium carbonate in acetone (2¹/2 days) using the method described in Example 1b. The crude product was chromatographed on silica gel eluting with chloroform/methanol (95:5) to afford the title compound (E7) as a white solid (10%) mp. 134-135°C.
¹H NMR (CDCl₃)
δ: 7.98 (d, 1H), 7.08-7.30 (m, 3H), 4.21 (d, 2H), 4.15 (s, 2H), 3.77 (s, 2H), 2.95-3.07 (m, 2H), 2.32-2.42 (m, 2H), 1.80-2.10 (m, 5H), 1.25-1.60 (m, 6H), 1.20 (s, 6H), 0.93 (t, 3H).
MS (Cl) MH⁺ 399.

### Example 8

### (1-ⁿButyl-4-piperidyl)methyl-3,4-dihydro-2H-[1,3]thiazino[3,2-a]indole-10-carboxylate (E8)

(1-ⁿButyl-4-piperidyl)methyl 1H-indole-3-carboxylate, Ela, (314mg, 0.0010 mole) was treated initially with N-chlorosuccinimide (180mg, 0.0015 mole) for 2h, then with 3-chloro-1-propanethiol (0.20ml, 0.0020 mole) for 5 days using the method described in Example 1b. The resulting solution was basified with 10% Na₂CO₃ solution and extracted with chloroform. The extract was dried (Na₂SO₄) and concentrated under vacuum to leave a dark oil which was chromatographed on silica gel eluting with chloroform/methanol (95:5) to afford (1-ⁿbutyl-4-piperidyl)methyl 2-(3-chloropropylmercapto)-1H-indole-3-carboxylate as a grey oil (220mg). This was dissolved in acetone (50ml), treated with anhydrous potassium carbonate (220mg, 0.0015 mole) and sodium iodide (390mg, 0.0026 mole) and heated under reflux for 8h. The mixture was concentrated under vacuum and the residue treated with 10% Na₂CO₃ solution, then extracted with ethyl acetate. The extract was dried (Na₂SO₄) and concentrated. The residue was chromatographed on basic alumina eluting with ethyl acetate. The colourless oil obtained crystallised from ether to afford the title compound (E8) as a white solid (80mg, 21%) mp 99-100°C.
¹H NMR (CDCl₃)
δ: 7.97-8.04 (m, 1H), 7.14-7.30 (m, 3H), 4.22 (d, 2H), 4.15 (t, 2H), 3.05-3.15 (m, 2H), 2.92-3.02 (m, 2H), 2.38-2.50 (m, 2H), 2.27-2.37 (m, 2H), 1.75-2.02 (m, 5H), 1.20-1.55 (m, 6H), 0.91 (t, 3H).
MS (El) M⁺ 386.

### Example 9

### (1-ⁿButyl-4-piperidyl)methyl-2,3,4,5-tetrahydro[1,3]oxazepino[3,2-a]indole-11-carboxylate (E9)

(1-ⁿButyl-4-piperidyl)methyl 1 H-indole-3-carboxylate (E1a) was treated initially with N-chlorosuccinimide (1.5 equivalents) for 2h, then with 4-chloro-1-butanol (2 equivalents) for 18h using the method of Example 1b and the product isolated as in Example 8 to afford (1-ⁿbutyl-4-piperidyl) methyl 2-(4-chlorobutoxy)-1H-indole-3-carboxylate as a yellow oil. A solution in acetone was treated with anhydrous potassium carbonate and sodium iodide and heated under reflux for 30h, then purified as in Example 8 to afford the title compound (E9) as a pale yellow oil (31%). This was converted to its oxalate salt and crystallised from acetone to give a white solid mp 161-164°C.
Oxalate salt:-¹H NMR (d⁶ DMSO)
δ: 7.85-7.95 (m, 1H), 7.45-7.55 (m, 1H), 7.10-7.25 (m, 2H), 4.15-4.30 (m, 4H), 4.10 (d, 2H), 3.35-3.45 (m, 2H), 2.80-3.05 (m, 4H), 1.80-2.10 (m, 7H), 1.50-1.75 (m, 4H), 1.20-1.40 (m, 2H), 0.89 (t, 3H).
MS (El) M⁺ 384.

### Example 10

### (1-ⁿButyl-4-piperidyl)methyl 6,7,8,9-tetrahydropyrido[1,2-a]indole-10-carboxylate (E10)

A solution of 6,7,8,9-tetrahydropyrido[1,2-a]indole-10-carboxylic acid, D3, (400mg, 0.00186 mole) in dichloromethane (20ml) was treated with oxalyl chloride (0.20ml, 0.0023 mole) and 2 drops of DMF and stirred at room temperature for 2h, then concentrated *in vacuo* to give the acid chloride as an orange solid.

A solution of (1-ⁿbutyl-4-piperidyl)methanol (D6) (0.32g, 0.00186 mole) in dry THF (25ml) at 5°C under nitrogen was treated with 1.5M methyllithium in ether (1.24ml, 0.00186 mole) and left to stir for 15 minutes, then treated with a solution of the above acid chloride in dry THF (15ml). After 16h at room temperature, the mixture was treated with saturated K₂CO₃ solution (50ml) and extracted into ethyl acetate (2x75ml), dried (Na₂SO₄) and concentrated *in vacuo.* The residue was chromatagraphed on silica gel eluting with chloroform/ethanol (95:5) to afford the title compound (E10) as a yellow oil. This was converted to its hydrochloride salt to afford a white solid. m.p. 230-232°C.
HCI salt: ¹H NMR (d⁶DMSO)
δ: 10.3 (br.s, 1H), 7.92-8.03 (m, 1H), 7.43-7.53 (m, 1H), 7.16-7.26 (m, 2H), 4.18 (d, 2H), 4.11 (t, 2H), 3.43-3.56 (m, 2H), 3.23 (t, 2H), 2.82-3.05 (m, 4H), 1.85-2.12 (m, 7H), 1.60-1.80 (m, 4H), 1.25-1.40 (m, 2H), 0.90 (t, 3H).
MS (El) M⁺ 368

### Example 11

### (1-ⁿButyl-4-piperidyl)methyl-2,3-dihydro-1H-pyrrolo[1,2-a]indole-9-carboxylate (E11)

The title compound (E11) was prepared from 2,3-dihydro-1H-pyrrolo[1,2-a]indole-9-carboxylic acid (D4) using the method of Example 10, and was isolated as a pale orange solid (24%) m.p. 100-102°C.
¹H NMR (CDCl₃)
δ: 8.03-8.12 (m, 1H), 7.13-7.28 (m, 3H), 4.17 (d, 2H), 4.11 (t, 2H), 3.29 (t, 2H), 2.95-3.08 (m, 2H), 2.57-2.72 (m, 2H), 2.30-2.41 (m, 2H), 1.92-2.07 (m, 2H), 1.73-1.90 (m, 3H), 1.40-1.60 (m, 4H), 1.22-1.39(m, 2H), 0.92 (t, 3H).
MS (El) M⁺ 354.

### Example 12

### (1-ⁿButyl-4-piperidyl)methyl 7,8,9,10-tetrahydro-6H-azepino[1,2-a]indole-11-carboxylate (E12)

The title compound (E12) was prepared from 7,8,9,10-tetrahydro-6H-azepino[1,2-a]indole-11-carboxylic acid (D5) using the method of Example 10. The crude product was purified by chromatography on silica gel eluting with chloroform/ethanol (98:2) to give a yellow oil, which was converted to its hydrochloride salt to afford a beige solid (20%) mp 196-198°C.
¹H NMR (d⁶DMSO) - HCl salt
δ: 10.52(brs, 1H), 7.93-8.00(m,1H), 7.55-7.62(m,1H), 7.13-7.25(m,2H), 4.25-4.40(m,2H), 4.17(d,2H), 3.35-3.55(m,4H), 2.80-3.10(m,4H), 1.55-2.15(m,13H), 1.24-1.40(m,2H), 0.88(t,3H).
MS (Cl) MH⁺ 383

### Example 13

### N-[(1-ⁿButyl-4-piperidyl)methyl]-2,3-dihydro-1H-pyrrolo[1,2-a]indole-9-carboxamide (E13)

A solution of 2,3-dihydro-1H-pyrrolo[1,2-a]indole-9-carboxylic acid, D4, (180mg, 0.89-mmole) in dichloromethane (20 ml) was treated with oxalyl chloride (0.096 ml, 1.1. mmole) and 2 drops of DMF and stirred at room temperature for 1h, then concentrated *in vacuo* to give the acid chloride as a yellow solid.

A solution of (1-ⁿbutyl-4-piperidyl)methylamine, D8, (150mg, 0.89 mmole) and triethylamine (0.15 ml, 1.1 mmole) in dichloromethane (20 ml) under nitrogen was treated with a solution of the above acid chloride in dichloromethane (5 ml) and stirred at room temperature for 3h. The solution was treated with 10% Na₂CO₃ solution and the organic layer separated, dried (Na₂SO₄) and concentrated *in vacuo* to leave a beige solid. This was recrystallised from ethyl acetate to afford the title compound (E13) as a white solid (180mg, 55%) mp 152-154°C.
¹H NMR (CDCl₃)
δ: 7.75-7.84(m,1H), 7.13-7.33(m,3H), 5.93(br t, NH), 4.10(t,2H), 3.38(t,2H), 3.31(t,2H), 2.90-3.02(m,2H), 2.65(quintet,2H), 2.28-2.36(m,2H), 1.60-2.10(m,6H), 1.22-1.55(m,5H), 0.90(t,3H).
MS (Cl) MH⁺ 354

### Example 14

### N-[(1-ⁿButyl-4-piperidyl)methyl]-2,3-dihydrooxazolo[3,2-a]indole-9-carboxamide (E14)

N-[(1-ⁿButyl-4-piperidyl)methyl]indole-3-carboxamide (D1) was treated initially with N-chlorosuccinimide (1.5 equivalents) for 2h, then with 2-bromoethanol (2 equivalents) for 16h, followed by potassium carbonate (3 equivalents) in acetone for 68h, using the method described in Example 1b. The crude product was purified by chromatography on silica gel eluting with chloroform/ethanol (19:1) to afford the title compound (E14) as a white solid following recrystallisation from chloroform/ether (14%) mp 156-158°C.
¹H NMR (CDCl₃)
δ: 8.19(d,1H), 7.00-7.30(m,3H), 6.00(t,NH), 5.15(t,2H), 4.20(t,2H), 3.32(t,2H), 2.90-3.15(m,2H), 2.25-2.42(m,2H), 1.20-2.05(m,11H), 0.90(t,3H).
MS(Cl) MH⁺ 356

### Example 15

### (1-Benzyl-4-piperidyl)methyl-3,4-dihydro-2H-[1,3]oxazino[3,2-a]indole-10-carboxylate (E15)

a) Indole-3-carboxylic acid was converted to its acid chloride and then reacted with 1-benzyl-4-piperidinemethanol (D7) using the method given in Example 1 a. The resulting orange oil was chromatographed on silica gel eluting with chloroform/ethanol (9:1) to afford (1 -benzyl-4-piperidyl)methyl indole-3-carboxylate as a yellow oil (88%)
   ¹H NMR (CDCl₃)
   δ: 9.24(s,1H), 8.12-8.20(m,1H), 7.81(d,1H), 7.20-7.45(m,8H), 4.20(d,2H), 3.53(s,2H), 2.90-3.04(m,2H), 1.73-2.10(m,5H), 1.36-1.58(m,2H).
b) (1-Benzyl-4-piperidyl)methyl indole-3-carboxylate was treated initially with N-chlorosuccinimide (1.5 equivalents) for 2h, then with 3-bromo-1-propanol (2 equivalents) for 16h, followed by anhydrous potassium carbonate in acetone, using the method described in Example 1b. The crude product was purified by chromatography on silica gel eluting with chloroform/ethanol (19:1) to afford the title compound (E15) as a beige solid following recrystallisation from chloroform/ether (47%) mp 158-160°C.
   ¹H NMR (CDCl₃)
   δ: 7.94-8.00(m,1H), 7.10-7.38(m,8H), 4.48-4.56(m,2H), 4.19(d,2H), 4.05-4.12(m,2H), 3.50(s,2H), 2.88-2.98(m,2H), 2.28-2.39(m,2H), 1.75-2.08(m,5H), 1.35-1.55(m,2H).
   MS (Cl) MH⁺ 405.

### Example 16

### (1-ⁿButyl-4-piperidyl)methyl-3,4-dihydro-1-oxo-2H-[1,3]thiazino[3,2-a]indole-10-carboxylate (E16)

A solution of (1-ⁿbutyl-4-piperidyl)methyl 3,4-dihydro-2H-[1,3]thiazino[3,2-a]indole-10-carboxylate (E8, 80mg, 0.21 mmole) in acetone (5ml) and water (5ml) was treated with sodium periodate (100mg, 0.46 mmole) and stirred at room temperature for 24h. The solution was then treated with saturated K₂CO₃ solution (10ml) and extracted using ethyl acetate (2x25ml). The extract was dried (Na₂SO₄) and concentrated in *vacuo* to leave a yellow oil, which was chromatographed on silica gel eluting with 5% methanol /chloroform. The colourless oil obtained crystallised from ether to give the title compound (E16) as a white solid (27mg, 32%) mp 130-135°C.
¹H NMR (CDCl₃)
6: 8.24 (d, 1H), 7.30-7.50 (m, 3H), 4.54 (dd, 1H), 4.22-4.38 (m, 2H), 4.05 (dt, 1H), 3.40 (dd, 1H), 3.21 (dq, 1H), 2.86-3.08 (m, 3H), 2.30-2.45 (m, 3H), 1.80-2.10 (m, 5H), 1.40-1.65 (m, 4H), 1.20-1.40 (m, 2H), 0.90 (t, 3H).
MS (Cl) MH⁺ 403.

### Example 17

### (1-ⁿButyl-4-piperidyl)methyl 6,7-dihydropyrido[1,2-a]indole-10-carboxylate (E17)

The title compound was prepared from 6,7-dihydropyrido[1,2-a]indiole-10-carboxylic acid (D8) using the method of Example 10, and chromatographed on silica gel eluting with ethyl acetate to give a yellow solid (18%) mp 62-62°C (n-pentane).
¹H NMR (CDCl₃)
δ: 8.10-8.17 (m, 1H), 7.42 (dt, 1H), 7.18-7.33 (m, 3H), 6.25-6.35 (m, 1H), 4.22 (d, 2H), 4.15 (t, 2H), 2.90-3.05 (m, 2H), 2.63-2.75 (m, 2H), 2.29-2.38 (m, 2H), 1.75-2.04 (m, 5H), 1.25-1.55 (m. 6H), 0.91 (t, 3H).
MS (El) M⁺366.

### Example 18

### (1-ⁿButyl-4-piperidyl)methyl pyrido[1,2-a]indole-10-carboxylate (E18)

The title compound was prepared from pyrido[1,2-a]indole-10-carboxylic acid (D9) using the method of Example 10 and chromatographed on silica gel eluting with ethyl acetate to give a yellow solid (10%) mp 57-59°C (n-pentane).
¹H NMR (CDCl₃)
δ: 8.35-8.50 (m, 3H), 7.88 (d, 1H), 7.48-7.56 (m, 1H), 7.28-7.40 (m, 2H), 6.78-6.86 (m, 1H), 4.30 (d, 2H), 2.95-3.05 (m, 2H), 2.30-2.40 (m, 2H), 1.85-2.05 (m, 5H), 1.43-1.60 (m, 4H), 1.25-1.40 (m, 2H), 0.92 (t, 3H).
MS (El) M⁺ 364.

### Example 19

### N-[(1-ⁿButyl-4-piperidyl)methyl] 3,4-dihydro-2H-[1,3]thiazino[3,2-a]indole-10-carboxamide (E19)

The title compound was prepared from N-[(1-ⁿbutyl-4-piperidyl)methyl] indole-3-carboxamide (D1b) using the method of Example 8 as a white solid (7%) mp 141-142°C.
¹H NMR (CDCl₃)
δ: 7.70(d,1H), 7.13-7.30(m,3H), 6.07(t,1H), 4.16(t,2H), 3.38(t,2H), 3.08(t,2H), 2.90-3.02(m,2H), 2.38-2.50(m,2H), 2.25-2.36(m,2H), 1.60-2.00(m,5H), 1.23-1.56(m,6H), 0.91(t,3H).
MS (El) M⁺ 385.

### Example 20

### N-[(1-Benzyl-4-piperidyl)methy]3,4-dihydro-2H-[1,3]oxazino[3,2-a]indole-10-carboxamide (E20)

a) indole-3-carboxylic acid was converted to its acid chloride and then reacted with (1-benzyl-4-piperidyl)methylamine (D10) as in the method of Description 1b to afford N-[(1-benzyl-4-piperidyl)methyl]indole-3-carboxamide as a white solid (60%).
   ¹H NMR (CDCl₃)
   δ : 9.90(s,1H), 7.85-7.95(m,1H), 7.64(d,1H), 7.15-7.43(m,8H), 6.17(t,1H), 3.48(s,2H), 3.37(t,2H), 2.83-2.98(m,2H), 1.87-2.08(m,2H), 1.54-1.82(m,3H), 1.23-1.50(m,2H).
b) A stirred suspension of N-[(1-benzyl-4-piperidyl)methyl] indole-3-carboxamide (17.5g, 0.050 mole) in chloroform (250 ml) was treated with 3-bromo-1-propanol (10.1 ml, 0.11 mole) and N-chlorosuccinimde (8.7g, 0.065 mole) at room temperature and a clear solution was obtained in 15 minutes. After 1h the reaction mixture darkened in colour from pale yellow to orange and temperature rose to 38°C. After a further 1 h the reaction mixture was treated with 10% NaHCO₃ solution and the chloroform layer separated, dried (Na₂SO₄) and concentrated *in vacuo* to leave a yellow oil, which was chromatographed on silica gel eluting with 3% methanol/chloroform. The 2-(3-bromopropoxy)indole intermediate was dissolved in acetone (400 ml), treated with anhydrous potassium carbonate (11g, 0.80 mole) and stirred at room temperature for 20h. The reaction mixture was concentrated *in vacuo* and the residue treated with water (200 ml) and extracted with chloroform (2 x 250 ml). The combined extracts were dried (Na₂SO₄), concentrated *in vacuo* and the residue chromatographed on silica gel eluting with 5% methanol/chloroform to afford the title compound (E20) as a pale yellow oil (3.1g, 15%). This was converted to its oxalate salt and crystallised from acetone as a white solid mp 169-170°C.
   Free base:- ¹H NMR (CDCl₃)
   δ: 8.32(d,1H), 7.05-7.38(m,8H), 6.53(t,1H), 4.50(t,2H), 4.08(t,2H), 3.48(s,2H), 3.31(t,2H), 2.83-2.97(m,2H), 2.27-2.41(m,2H), 1.54-2.06(m,5H), 1.25-1.45(m,2H).

### Example 21

### N-(4-Piperidylmethyl) 3,4-dihydro-2H-[1,3]oxazino[3,2-a]indole-10-carboxamide (E21)

A stirred suspension of N-[(1-benzyl-4-piperidyl)methyl] 3,4-dihydro-2H-[1,3]oxazino[3,2-a]indole-10-carboxamide oxalate salt (E20, 2.25g, 0.0046 mole) in ethanol (100 ml) and glacial acetic acid (4 ml) was hydrogenated over 10% Pd-C (0.8g) at atmospheric pressure and 45°C for 18h. The mixture was filtered and the filtrate concentrated *in vacuo.* The majority of the product was in the solid which had been filtered off. This material was shaken with concentrated potassium carbonate solution (50 ml) and chloroform (50 ml) together with the residue from the filtrate. The mixture was filtered, the chloroform layer separated and dried (Na₂SO₄), then concentrated *in vacuo* to afford the title compound as a white solid (1.52g, 100%). This was recrystallised from chloroform/60-80 petrol mp 139-141°C.
¹H NMR (CDCl₃)
δ: 8.32(d,1H), 7.03-7.30(m,3H), 6.53(t,1H), 4.48(t,2H), 4.05(t,2H), 3.30(t,2H), 3.02-3.15(m,2H), 2.52-2.70(m,2H), 2.27-2.40(m,2H), 1.65-1.90(m,4H), 1.10-1.30(m,2H).
MS (El) M⁺ 313.

### Example 22

### N-[(1-ⁿHexyl-4-piperidyl)methyl] 3,4-dihydro-2H-[1,3]oxazino[3,2-a]indole-10-carboxamide (E22)

A solution of N-(4-piperidylmethyl) 3,4-dihydro-2H-[1,3]oxazino[3,2-a]indole-10-carboxamide (E21, 250mg, 0.70 mmole) in acetone (12 ml) was treated with 1-bromohexane (0.14 ml, 1.0 mmole) and anhydrous potassium carbonate (280mg, 2.0 mmole) and stirred at room temperature for 70h. The mixture was concentrated *in vacuo* and the residue treated with 10% Na₂CO₃ solution and extracted with chloroform. The extract was dried (Na₂SO₄), concentrated *in vacuo* and the residue chromatographed on silica gel eluting with 5% methanol/chloroform to give a yellow oil. This was passed through a short plug of basic alumina eluting with ethyl acetate to afford the title compound (E22) as a colourless oil (150mg, 54%). This was converted to its hydrochloride salt and crystallised from acetone/ether as a white solid mp 170-171°C.
Free base:- ¹H NMR (CDCl₃)
δ: 8.32(d,1H), 7.02-7.30(m,3H), 6.53(t,1H), 4.48(t,2H), 4.04(t,2H), 3.32(t,2H), 2.90-3.00(m,2H), 2.25-2.38(m,4H), 1.83-1.96(m,2H), 1.20-1.81(m,13H), 0.88(t,3H).
MS (El) M⁺ 397.

### Example 23

### N-[(1-Cyclohexylmethyl-4-piperidyl)methyl]3,4-dihydro-2H-[1,3]oxazino[3,2-a]indole-10-carboxamide (E23)

N-(4-Piperidylmethyl) 3,4-dihydro-2H-[1 ,3]oxazino[3,2-a]indole-10-carboxamide (E21) was alkylated with cyclohexylmethyl bromide using the method of Example 22 with a reaction time of 70h at room temperature followed by 8h at reflux temperature. The title compound (E23) was obtained as a white solid (31%) which was converted to its hydrochloride salt and crystallised from acetone/ether as a white solid mp 209-210°C.
HCl salt:- ¹H NMR (CD₃OD)
δ: 8.03-8.09(m,1H), 7.20-7.28(m,1H), 7.10-7.17(m,2H), 4.60(t,2H), 4.15(t,2H), 3.53-3.65(m,2H), 3.36(d,2H), 2.85-3.05(m,4H), 2.30-2.43(m,2H), 1.50-2.07(m,11H), 1.18-1.46(m,3H), 0.95-1.13(m,2H).
MS (El) M⁺ 409.

### Example 24

### N-[(1-Ethyl-4-piperidyl)methyl] 3,4-dihydro-2H-[1,3]oxazino[3,2-a]indole-10-carboxamide (E24)

N-(4-Piperidylmethyl) 3,4-dihydro-2H-[1 ,3]oxazino[3,2-a]indole-10-carboxamide (E21) was alkylated with iodoethane using the method of Example 22. The title compound was obtained as a white solid (27%), which was converted to its hydrochloride salt and crystallised from acetone/ethanol/ether as a white solid mp 243-245°C.
Free base:- ¹H NMR (CDCl₃)
δ: 8.34(d,1H), 7.05-7.28(m,3H), 6.55(t,1H), 4.52(t,2H), 4.07(t,2H), 3.33(t,2H), 2.90-3.02(m,2H), 2.30-2.40(m,4H), 1.55-1.98(m,5H), 1.25-1.45(m,2H), 1.08(t,3H).
MS (El) M⁺ 341.

### Example 25

### N-[(1-(2-Methanesulphonamidoethyl)-4-piperidyl)methyl] 3,4-dihydro-2H-[1,3]oxazino[3,2-a]indole-10-carboxamide (E25)

A stirred solution of N-(4-piperidylmethyl) 3,4-dihydro-2H-[1,3]oxazino[3,2-a]indole-10-carboxamide (E21, 220mg, 0.70 mmole) in acetonitrile (8 ml) was treated with diisopropylethylamine (0.24 ml, 1.4 mmole) and N-(2-bromoethyl)methanesulphonamide (D14, 160mg, 0.77 mmole) and the mixture heated under reflux for 2.5h. The reaction mixture was concentrated *in vacuo* and the residue chromatographed on silica gel eluting with dichloromethane/methanol/0.88 ammonia solution (200:10:1). The colourless oil obtained was dissolved in chloroform (30 ml) and washed with water (2 x 20 ml), then dried (Na₂SO₄) and concentrated *in vacuo.* The residue was passed through a short plug of basic alumina eluting with ethyl acetate to afford the title compound as a colourless oil (34mg, 11%). This was converted to its oxalate salt and crystallised from acetone to give a white solid mp 80-85°C.
Free base:- ¹H NMR (CDCl₃)
δ: 8.32(d,1H), 7.05-7.30(m,3H), 6.56(t,1H), 4.53(t,2H), 4.08(t,2H), 3.33(t,2H), 3.17(t,2H), 2.95(s,3H), 2.78-2.92(m,2H), 2.50(t,2H), 2.28-2.44(m,2H), 1.55-2.10(m,6H), 1.20-1.45(m,2H).

### Example 26

### N-(eq-Quinolizidin-2-ylmethyl) 3,4-dihydro-2H-[1,3]oxazino[3,2-a]indole-10-carboxamide (E26)

a) *eq*-Quinolizidin-2-ylmethylamine (D12) was reacted with indole-3-carboxylic acid chloride using the method of Description 1b) to afford N-(*eq-*quinolizidin-2-ylmethyl) indole-3-carboxamide as a white solid (55%).
   ¹H NMR (CD₃OD)
   δ: 8.06-8.15(m,1H), 7.89(s,1H), 7.39-7.46(m,1H), 7.10-7.22(m,2H), 3.27(d,2H), 2.80-2.95(m,2H), 2.04-2.23(m,2H), 1.53-1.98(m,8H), 1.22-1.48(m,3H), 0.96-1.15(m,1H).
b) A stirred suspension of N-(*eq*-quinolizidin-2-ylmethyl) indole-3-carboxamide (300mg, 0.94 mmole) in chloroform (16 ml) was treated with 3-bromo-1-propanol (0.17 ml, 1.9 mmole) followed by N-chlorosuccinimide (140mg, 1.05 mmole) and gave a clear solution inside 30 minutes. After 2h the mixture was treated with 1M HCl/ether (3 drops) giving a yellow colouration, then after 1.5h the mixture was treated with excess 10% NaHCO₃ solution and the chloroform layer separated, dried (Na₂SO₄) and concentrated *in vacuo* to leave a yellow oil. This was dissolved in acetone (20 ml), treated with anhydrous potassium carbonate (400mg, 2.9 mmole) and stirred at room temperature for 24h, then concentrated *in vacuo.* The residue was treated with 10% Na₂CO₃ solution and extracted with chloroform. The extract was dried (Na₂SO₄) and concentrated to leave a yellow oil, which was chromatographed on silica gel eluting with 10% methanol/chloroform. The oil obtained was passed through a short plug of basic alumina eluting with ethyl acetate to afford the title compound (E26) as a colourless oil (110mg, 32%). This was converted to its hydrochloride salt and crystallised from methanol/ether as a white solid mp 243-247°C.
   Free base:- ¹H NMR (CDCl₃)
   δ: 8.30(d,1H), 6.98-7.25(m,3H), 6.51(t,1H), 4.45(t,2H), 3.96(t,2H), 3.20-3.37(m,2H), 2.78-2.92(m,2H), 2.20-2.35(m,2H), 1.94-2.14(m,2H), 0.98-1.85(m,12H).
   MS (Cl) MH⁺ 368.

### Example 27

### (1-ⁿButyl-4-piperidyl)methyl 8-fluoro-3,4-dihydro-2H-[1,3]oxazino[3,2-a]indole-10-carboxylate (E27)

a) 5-Fluoroindole-3-carboxylic acid chloride was reacted with (1-ⁿbutyl-4-piperidyl)methanol (D6) using the method of Example 1a to afford (1-ⁿbutyl-4-piperidyl)methyl 5-fluoroindole-3-carboxylate as an orange oil (30%), following flash chromatography on silica gel eluting with 10% ethanol/chloroform.
   ¹H NMR (CDCl₃)
   δ: 9.95(br s,1H), 7.82(s,1H), 7.78(dd,1H), 7.33(dd,1H), 7.00(dt,1H), 4.22(d,2H), 3.00-3.15(m,2H), 2.33-2.47(m,2H), 1.95-2.10(m,2H), 1.75-1.93(m,3H), 1.22-1.65(m,6H), 0.92(t,3H).
b) (1-ⁿButyl-4-piperidyl)methyl 5-fluoroindole-3-carboxylate was reacted with N-chlorosuccinimide and 3-bromo-1-propanol, then with potassium carbonate in acetone using the method of Example 26b to give a pale oil, which was flash chromatographed on silica gel eluting with 10% ethanol/chloroform. This afforded the title compound (E27) as a pale yellow oil (8%), which was converted to its oxalate salt and obtained as a beige solid mp 118-119°C.
   Free base:- ¹H NMR (CDCl₃)
   δ: 7.64(dd,1H), 7.04(dd,1H), 6.87(dt,1H), 4.55(t,2H), 4.20(d,2H), 4.10(t,2H), 2.96-3.10(m,2H), 2.28-2.47(m,4H), 1.77-2.14(m,5H), 1.25-1.65(m,6H), 0.92(t,3H).
   MS (Cl) MH⁺ 389.

### Example 28

### N-[(1-ⁿButyl-4-piperidyl)methyl]8-fluoro-3,4-dihydro-2H-[1,3]oxazino[3,2-a]indole-10-carboxamide (E28)

a) 5-Fluoroindole-3-carboxylic acid chloride was reacted with (1-ⁿbutyl-4-piperidyl)methylamine (D1a) as in the method of Description 1b to afford N-[(1-ⁿbutyl-4-piperidyl)methyl] 5-fluoroindole-3-carboxamide as an off-white solid (64%).
   ¹H NMR (CD₃OD)
   δ: 7.92(s,1H), 7.78(dd,1H), 7.38(dd,1H), 6.95(dt,1H), 3.28(d,2H), 2.93-3.07(m,2H), 2.30-2.42(m,2H), 1.60-1.87(m,3H), 1.22-1.60(m,6H), 0.94(t,3H).
b) N-[(1-ⁿButyl-4-piperidyl)methyl]5-fluoroindole-3-carboxamide was reacted with 3-bromo-1-propanol and N-chlorosuccinimide, then with potassium carbonate in acetone using the method of Example 26b to give a yellow oil, which was flash chromatographed on silica gel eluting with 20% ethanol/chloroform to afford the title compound as a pale yellow oil (8%). This was converted to its hydrochloride salt, which was obtained as a beige solid mp 90°C dec.
   Free base:- ¹H NMR (CDCl₃)
   δ: 7.98(dd,1H), 6.98(dd,1H), 6.83(dt,1H), 6.56(t,1H), 4.56(t,2H), 4.08(t,2H), 3.33(t,2H), 3.05-3.20(m,2H), 2.30-2.58(m,4H), 2.10-2.26(m,2H), 1.25-1.90(m,9H), 0.92(t,3H).
   MS (Cl) MH⁺ 388.

### Example 29

### (1-ⁿButyl-4-piperidyl)methyl 1-methyl-1,2,3,4-tetrahydropyrimido[1,2-a]indole-10-carboxylate (E29)

A solution of (1-ⁿbutyl-4-piperidyl)methanol (D6, 1.7g, 0.010 mole) in dry THF (20 ml) under argon at 10°C was treated with 1.5M methyllithium in ether (2.7 ml, 0.004 mole) and stirred for 15 minutes, then a solution of methyl 1-methyl-1,2,3,4-tetrahydropyrimido[1,2-a]indole-10-carboxylate (D11, 0.5g, 0.002 mole) in THF (5 ml) was added and the reaction mixture heated under reflux for 24h. The mixture was allowed to cool and then treated with 10% Na₂CO₃ solution (50 ml) and extracted with ethyl acetate (2 x 40 ml). The combined extracts were dried (Na₂SO₄), concentrated *in vacuo* and the residue chromatographed on silica gel eluting with 2% methanol/chloroform to afford the title compound (E29) as a colourless oil (0.58g, 74%). This was converted to its oxalate salt and recrystallised from methanol to afford a white solid mp 186-187°C.
Free base:- ¹H NMR (CDCl₃)
δ: 7.92(d,1H), 7.00-7.20(m,3H), 4.17(d,2H), 3.95(t,2H), 3.37(t,2H), 3.28(s,3H), 2.92-3.03(m,2H), 2.28-2.38(m,2H), 2.12-2.24(m,2H), 1.80-2.03(m,5H), 1.23-1.57(m,6H), 0.92(t,3H).
MS (El) M⁺ 383.

### Example 30

### (1-ⁿButyl-4-piperidyl)methyl 3-methylthiazolo[3,2-a]indole-9-carboxylate (E30)

The title compound (E30) was prepared from 3-methylthiazolo[3,2-a]indole-9-carboxylic acid (D13) using the method of Example 10. The crude product was purified by chromatography on silica gel eluting with chloroform/methanol (95:5), followed by passage through a short plug of basic alumina eluting with ether to afford a pale yellow oil (35%). This was converted to its oxalate salt and crystallised from methanol to give a white solid mp 224-226°C.
Free base:- ¹H NMR (CDCl₃)
δ: 8.18(d,1H), 7.77(d,1H), 7.14-7.42(m,2H), 6.40(s,1H), 4.25(d,2H), 2.92-3.08(m,2H), 2.73(s,3H), 2.28-2.40(m,2H), 1.75-2.05(m,5H), 1.20-1.62(m,6H), 0.92(t,3H).
MS (Cl) MH⁺ 385.

### Example 31

### (1-ⁿButyl-4-piperidyl)methyl-2,3-dihydrothiazolo[3,2-a]indole-9-carboxylate (E31)

The title compound was prepared from 2,3-dihydrothiazolo[3,2-a]indole-9-carboxylic acid (D15) using the method of Example 10. The crude product was purified by chromatography on silica gel eluting with 5% methanol/chloroform to give a yellow oil. This was passed through a plug of basic alumina eluting with ethyl acetate to afford the title compound as a pale yellow oil (31%) which was converted to its oxalate salt and was crystallised from acetone as an off-white solid mp 212-215°C.
Free base:- ¹H NMR (CDCl₃)
δ: 7.98(d,1H), 7.09-7.26(m,3H), 4.26(t,2H), 4.20(d,2H), 3.80(t,2H), 2.94-3.06(m,2H), 2.30-2.40(m,2H), 1.73-2.06(m,5H), 1.24-1.60(m,6H), 0.92(t,3H).

### Example 32

### (1-ⁿButyl-4-piperidyl)methyl thiazolo[3,2-a]indole-9-carboxylate (E32)

The title compound (E32) was prepared from thiazolo[3,2-a]indole-9-carboxylic acid (D16) using the method of Example 10. The crude product was purified by chromatography on silica gel eluting with 3%methanol/chloroform to afford a pale purple solid (70%). This was converted to its oxalate salt and recrystallised from methanol to give a light blue solid mp 217-218°C.
Free base:- ¹H NMR (CDCl₃)
δ: 8.18(d,1H), 7.79(d,1H), 7.65(d,1H), 7.33-7.43(m,1H), 7.20-7.30(m,1H), 6.91(d,1H), 4.27(d,2H), 2.95-3.07(m,2H), 2.30-2.40(m,2H), 1.79-2.08(m,5H), 1.40-1.62(m,4H), 1.33(sextet,2H), 0.92(t,3H).

### Example 33

### (1-ⁿButyl-4-piperidyl)methyl 2,4-dimethylpyrimido[1,2-a]indole-10-carboxylate (E33)

The title compound (E33) was prepared from methyl 2,4-dimethylpyrimido[1,2-a]indole-10-carboxylate (D17) using the method of Example 29. The crude product was purified by chromatography on silica gel eluting with ethyl acetate to afford an orange oil (21%). This was converted to its oxalate salt to give an orange solid mp 195-198°C.
Oxalate salt:- ¹H NMR (d⁶DMSO)
δ: 8.45(d,1H), 8.35(d,1H), 7.59(t,1H), 7.41(t,1H), 6.97(s,1H), 4.90(br s,2H), 4.27(d,2H), 3.38-3.60(m,2H), 3.14(s,3H), 3.27-3.04(m,4H), 2.61(s,3H), 2.01-2.27(m,3H), 1.55-1.84(m,4H), 1.37(sextet,2H), 0.97(t,3H).

### Example 34

### N-[(1-ⁿButyl-4-piperidyl)methyl] 2,3-dihydrothiazolo[3,2-a]indole-9-carboxamide

The title compound (E34) was prepared from 2,3-dihydrothiazolo[3,2-a]indole-9-carboxylic acid (D15) *via* its acid chloride using the method of Example 13. The crude product was purified by chromatography on silica gel eluting with 5% methanol/chloroform to afford a yellow solid (63%). This was converted to its oxalate salt and recrystallised from acetone to give a beige solid mp 203-204°C.
Oxalate salt:- ¹H NMR (d⁶DMSO)
δ: 7.83-7.92(m,1H), 7.33-7.45(m,2H), 7.08-7.18(m,2H), 4.35(t,2H), 3.84(t,2H), 3.35-3.50(m,2H), 3.18-3.30(m,2H), 2.75-3.05(m,4H), 1.75-1.95(m,3H), 1.40-1.70(m,4H), 1.30(sextet,2H), 0.88(t,3H).

### Example 35

### N-[1-ⁿButyl-4-piperidyl)methyl]thiazolo[3,2-a]indole-9-carboxamide (E35)

The title compound (E35) was prepared from thiazolo[3,2-a]indole-9-carboxylic acid (D16) via its acid chloride using the method of Example 13. The crude product was purified by chromatography on silica gel eluting with 5% methanol/chloroform to afford a purple solid (73%). This was converted to its oxalate salt and recrystallised from acetone to give a purple solid mp 205-207°C.
Oxalate salt:- ¹H NMR (d⁶DMSO)
δ: 8.49(d,1H), 8.14(d,1H), 8.05(d,1H), 7.54(t,1H), 7.20-7.40(m,3H), 3.38-3.50(m,2H), 3.24-3.35(m,2H), 2.75-3.05(m,4H), 1.80-2.00(m,3H), 1.40-1.70(m,4H), 1.30(sextet,2H), 0.88(t,3H).

### Example 36

### (1-ⁿButyl-4-piperidyl)methyl 1,2,3,4-tetrahydropyrimido[1,2-a]indole-10-carboxylate (E36)

The title compound (E36) was prepared from methyl 1,2,3,4-tetrahydropyrimido[1,2-a]indole-10-carboxylate (D21) using the method of Example 29 with a relux time of 140h. The crude product was purified by chromatography on silica gel eluting initially with ethyl acetate, then with 10% methanol/ethyl acetate to give a yellow solid. This was passed through a plug of basic alumina eluting with ethyl acetate to afford the title compound as a beige solid (23%), which was converted to its oxalate salt and crystallised from acetone as a beige solid mp 190-194°C.
Free base:- ¹H NMR (CDCl₃)
δ: 7.71(br d,1H), 6.98-7.18(m,3H), 7.0(br s,1H), 4.17(d,2H), 3.98(t,2H), 3.46-3.57(m,2H), 2.92-3.06(m,2H), 2.30-2.40(m,2H), 2.22(quintet,2H), 1.75-2.08(m,5H), 1.23-1.60(m,6H), 0.92(t,3H).

### Example 37

### eq-Quinolizidin-2-ylmethyl 2,3-dihydrooxazolo[3,2-a]indole-9-carboxylate(E37)

A stirred suspension of *eq*-quinolizidin-2-ylmethyl 1 H-indole-3-carboxylate (E2a, 280mg, 0.94 mmole) in chloroform (10 ml) was treated with 2-bromoethanol (0.13 ml) followed by N-chlorosuccinimide (135mg, 1.0 mmole) and kept at room temperature for 2h. The mixture was then treated with 1M HCI in ether (0.05 ml, 0.05 mmole) and after 2h the resulting yellow solution was basified by addition of 10% Na₂CO₃ solution (10 ml) and extracted with chloroform (2 x 15 ml). The combined extracts were dried (Na₂SO₄) and concentrated *in vacuo* to leave an orange oil. This was dissolved in acetone (20 ml), treated with anhydrous potassium carbonate (410mg, 3.0 mmole) and stirred at room temperature for 22h, then concentrated *in vacuo* and the residue treated with 10% Na₂CO₃ solution (20 ml) and extracted with ethyl acetate (2 x 20 ml). The combined extracts were dried (Na₂SO₄), concentrated *in vacuo* and the residiue chromatographed on silica gel eluting with 3% methanol/chloroform. The yellow oil obtained (145mg, 44%) was passed through a plug of basic alumina eluting with ethyl acetate to afford the title compound (E37) which crystallised as a white solid from ethyl acetate/ether mp 153-155°C .
¹H NMR (CDCl₃)
δ: 7.95(d,1H), 7.00-7.25(m,3H), 5.14(t,2H), 4.18(t,2H), 4.15(d,2H), 2.78-2.96(m,2H), 1.02-2.18(m,14H).

### Example 38

### N-[(1-ⁿButyl-4-piperidyl)methyl] 2,3,4,5-tetrahydro[1,3]oxazepino[3,2-a]indole-11-carboxamide (E38)

a) A stirred suspension of N-[(1-ⁿbutyl-4-piperidyl)methyl] indole-3-carboxamide (D1b, 1.0g, 0.0032 mole) in chloroform (25 ml) was treated with 4-chlorobutanol (0.69 ml, 0.0064 mole) followed by N-chlorosuccinimide (470mg, 0.0035 mole) and a yellow solution was produced inside 5 minutes. After a further 40 minutes the solution was observed to darken in colour to orange. The mixture was kept at room temperature for a further 1h then treated with 10% Na₂CO₃ solution (30 ml) and extracted with chloroform (2 x 30 ml). The combined extracts were dried (Na₂SO₄) and concentrated *in vacuo* to afford an orange oil, which was chromatographed on silica gel eluting with 5% methanol/chloroform to give N-[(1-ⁿ-butyl-4-piperidyl)methyl] 2-(4-chlorobutoxy)indole-3-carboxamide (0.67g, 50%) as a yellow oil.
   ¹H NMR (CDCl₃)
   δ:10.7(br s,1H), 8.23(d,1H), 7.00-7.32(m,3H), 6.88(t,1H), 4.43(t,2H), 3.48(t,2H), 3.34(t,2H), 2.86-3.02(m,2H), 2.25-2.40(m,2H), 1.18-2.00(m,15H), 0.90(t,3H).
b) A solution of N-[(1-ⁿbutyl-4-piperidyl)methyl] 2-(4-chlorobutoxy)indole-3-carboxamide (0.67g, 0.0016 mole) in acetone (25 ml) was treated with anhydrous potassium carbonate (0.74g, 0.0054 mole) and sodium iodide (1.34g, 0.0089 mole) and heated under refux for 24h. The mixture was concentrated *in vacuo* and the residue treated with 10% Na₂CO₃ solution (25 ml) and extracted with chloroform (2 x 30 ml). The combined extracts were dried (Na₂SO₄), concentrated *in vacuo* and the residue chromatographed on silica gel eluting with 5% methanol/chloroform. The colourless oil obtained was passed through a plug of basic alumina eluting with ethyl acetate to afford the title compound (E38) as a white solid (370mg, 60%). This was converted to its oxalate salt and crystallised from acetone as a white solid mp 210-211°C.
   Free base:- ¹H NMR (CDCl₃)
   δ: 8.36-8.44(m,1H), 7.17-7.25(m,3H), 6.94(t,1H), 4.30(t,2H), 4.11-4.20(m,2H), 3.35(t,2H), 2.90-3.00(m,2H), 2.25-2.35(m,2H), 2.18(quintet,2H), 1.55-2.02(m,7H), 1.23-1.55(m,6H), 0.92(t,3H).

### Example 39

### (1-ⁿButyl-4-piperidyl)methyl pyrimido[1,2-a]indole-10-carboxylate (E39)

The title compound was prepared from methyl pyrimido[1,2-a]indole-10-carboxylate (D19) using the method of Example 29. The crude product was washed at -78°C with n-pentane and the residue chromatographed on silica gel eluting with 5% methanol/chloroform to afford an orange oil.
¹H NMR (CDCl₃)
δ: 8.68-8.78(m,2H), 8.45(d,1H), 7.87(d,1 H), 7.59(t,1 H), 7.45(t,1H), 6.77-6.89(m,1H), 4.37(d,2H), 2.90-3.12(m,2H), 2.25-2.48(m,2H), 1.75-2.13(m,5H), 1.19-1.70(m,6H), 0.92(t,3H).

The following compounds are also prepared:

### eq-Quinolizidin-2-ylmethyl 2,3-dihydrothiazolo[3,2-a]indole-9-carboxylate (E40)

2,3-Dihydrothiazalo[3,2-a]indole-9-carboxylic acid is converted to its acid chloride and reacted with *eq*-2-hydroxymethylquinolizidine using a procedure analogous to that described in Example 10.
Free base:- ¹H NMR (CDCl₃)
δ: 8.00(d,1H), 7.15-7.30(m,3H), 4.34(t,2H), 4.10-4.25(m,2H), 3.87(t,2H), 2.80-3.00(m,2H), 1.05-2.20(m,14H).

### eq-Quinolizidin-2-ylmethyl 2,3-dihydrothiazolo[3,2-a]indole-9-carboxamide (E41)

2,3-Dihydrothiazolo[3,2-a]indole-9-carboxylic acid is converted to its acid chloride and reacted with eq-quinolizidin-2-ylmethylamine (D13) using a procedure analogous to that described in Description 1 b.

### eq-Quinolizidin-2-ylmethyl thiazolo[3,2-a]indole-9-carboxylate (E42)

Thiazolo[3,2-a]indole-9-carboxylic acid is converted to its acid chloride and reacted with *eq*-2-hydroxymethylquinolizidine using a procedure analogous to that described in Example 10 to afford the title compound as a white solid mp 129-131°C (ether).
¹H NMR (CDCl₃)
δ: 8.16(d,1H), 7.75(d,1H), 7.61(d,1H), 7.33-7.42(m,1H), 7.19-7.30(m,1H), 6.87(d,1H), 4.15-4.32(m,2H), 2.80-3.00(m,2H), 1.40-2.18(m,11H), 1.08-1.40(m,3H).

### eq-Quinolizidin-2-ylmethyl thiazolo[3,2-a]indole-9-carboxamide (E43)

Thiazolo[3,2-a]indole-9-carboxylic acid is converted to its acid chloride and reacted with *eq*-quinolizidin-2-ylmethylamine using a procedure analogous to that described in Description 1b.

### eq-Quinolizidin-2-ylmethyl 3,4-dihydro-2H-[1,3]thiazino[3,2-a]indole-10-carboxylate (E44)

3,4-Dihydro-2H-[1,3]thiazino[3,2-a]indole-10-carboxylic acid is prepared from thioxindole using a procedure analogous to that described in Description 15. This is converted to its acid chloride and reacted with *eq*-2-hydroxymethylquinolizidine using a procedure analogous to that described in Example 10. Oxalate salt mp 130-132°C.
Free base:- ¹H NMR (CDCl₃)
δ: 7.96-8.04 (m,1H), 7.13-7.30(m,3H), 4.05-4.30(m,4H), 2.90-3.20(m,4H), 2.35-2.51(m,2H), 1.20-2.32 (m,14H).

### (1-ⁿButyl-4-piperidyl)methyl pyrimido[1,2-a]indole-10-carboxamide (E45)

a) Benzyl pyrimido[1,2-a]indole-10-carboxylate is prepared using a procedure analogous to that described in Description 19 and then hydrogenated over 10% Pd/C in ethanol to afford pyrimido[1,2-a]indole-10-carboxylic acid.
b) Pyrimido[1,2-a]indole-10-carboxylic acid is converted to its acid chloride and reacted with (1-ⁿbutyl-4-piperidyl)methylamine (D1) using the procedure of Description 1b.

### (1-ⁿButyl-4-piperidyl)methyl 1,2,3,4-tetrahydropyrimido[1,2-a]indole-10-carboxamide (E46)

a) 2-Chloroindole-3-carboxylic acid (L. Marchetti and A Andreani, Ann. Chim. (Rome), 1973, 63, 681) was converted to its acid achloride and reacted with N-(1-ⁿbutyl-4-piperidyl)methylamine (D1) using the procedure of Description 1b to afford N-[(1ⁿbutyl-4-piperidyl)methyl] 2-chloroindole-3-carboxamide.
b) N-[(1-Butyl-4-piperidyl)methyl] 2-chloroindole-3-carboxamide is reacted with 3-chloropropylamine using a procedure analogous to that described in Description 18.

### Descriptions

### Description 1 (intermediates for Examples 3, 13, 14, 19 and 28)

### a) N-(1-ⁿButyl-4-piperidyl)methylamine

A stirred solution of isonipecotamide (70g, 0.55 mole) and 1-bromobutane (58.8 ml, 0.55 mole) in ethanol (700 ml) was treated with anhydrous potassium carbonate (152g, 1.10 mole) and heated under reflux for 3h. The mixture was allowed to cool, then filtered and the filtrate concentrated under vacuum. The residual oil was dissolved in chloroform (400 ml) and washed with water (1 x 300 ml), then dried (Na₂SO₄) and concentrated under vacuum to leave a yellow oil (77.5g). This oil was mixed thoroughly with phosphorus pentoxide (75g) and the mixture heated at 160-180°C under nitrogen for 2.5h with gentle stirring. The reaction mixture was allowed to cool, then treated with water (500 ml). When the solid mass had dissolved, the solution was basified by addition of solid K₂CO₃ and extracted with ethyl acetate (2x400 ml). The combined extracts were dried (Na₂SO₄) and concentrated *in vacuo* to leave a brown oil (78g). This was dissolved in dry ether (400 ml) and added dropwise over 30 minutes to a stirred suspension of lithium aluminium hydride (25g, 0.66 mole) in ether (200ml) at 0°C under nitrogen. When addition was complete, the mixture was allowed to warm upto room temperature and stir for 18h. It was re-cooled to 0°C and treated cautiously with water (25ml), 10% NaOH solution (25 ml) and water again (75ml). The mixture was filtered through kieselguhr and the filtrate concentrated *in vacuo* to leave a brown oil, which was distilled under vacuum to afford the title compound as a colourless oil (66g, 71%) bp 96-99°C at 3 mm Hg.
¹H NMR (CDCl₃)
δ: 2.90-3.02(m,2H), 2.58(d,2H), 2.25-2.38(m,2H), 1.65-2.00(m,4H), 1.08-1.58(m,9H), 0.92(t,3H).

### b) N-[(1-ⁿButyl-4-piperidyl)methyl] indole-3-carboxamide

To a stirring solution of indole-3-carboxylic acid (1g) in dichloromethane (20 ml) at 0°C under nitrogen was added oxalyl chloride (0.81 ml) and dry dimethylformamide (3 drops). After 3 hours, the solvents were evaporated under reduced pressure. A portion of the residual acid chloride (420 mg) was dissolved in dichloromethane (12 ml) and added dropwise to a solution of N-(1-ⁿbutyl-4-piperidyl)methylamine (400 mg) in dichloromethane (12 ml) followed by triethylamine (0.36 ml). After stirring at ambient temperature overnight, the reaction mixture was washed with saturated NaHCO₃, and the organic phase was dried (Na₂SO₄). The solvent was evaporated under reduced pressure and the residue recrystallised from ethyl acetate to give the title compound (D1) (467 mg, 64%).
¹H NMR (CDCl₃) 250 MHz
δ: 9.29 (br s,1H), 8.05-7.9 (m,1H), 7.81 (d,1H), 7.55-7.4 (m,1H), 7.39-7.2 (m,2H), 6.28 (br s,1H), 3.39 (t,2H), 3.0 (br d,2H), 2.45-2.25 (m,2H), 2.1-1.1 (m,11H), 0.9 (t,3H).

### Description 2 (intermediate for Example 5)

### N-[2-(1-Piperidyl)ethyl]1H-indole-3-carboxamide

1-Piperidineethylamine was reacted with 1 H-indole-3-carboxylic acid chloride using the method described in Description 1 to afford the title compound (D2) as a beige solid.
¹H NMR (CDCl₃)
δ: 9.90 (br.s, 1H), 7.97-8.07 (m, 1H), 7.78 (d, 1H), 7.36-7.50 (m, 1H), 7.15-7.30 (m, 2H), 7.13 (br.t, NH), 3.55-3.68 (m, 2H), 2.60 (t,2H), 2.40-2.55 (m, 4H), 1.40-1.73 (m, 6H).

### Description 3 (intermediate for Example 10)

a) **Ethyl 2-aminophenylacetate**
   A solution of ethyl 2-nitrophenylacetate (13.6g, 0.065 mole) in ethanol (150ml) was hydrogenated over 10% Pd/C catalyst (1g) at room temperature and pressure for 18 hours. The reaction mixture was filtered through keiselguhr and concentrated *in vacuo* to afford the title compound as a clear oil, which solidified on standing (10.8g, 93%).
   ¹H NMR (CDCl₃)
   δ: 7.05-7.15 (m, 2H), 6.68-6.80 (m, 2H), 4.13 (q, 2H), 4.05 (br.s, 2H), 3.55 (s, 2H), 1.25 (t, 3H).
**b) Ethyl 2-(5-chlorovalerylamino)phenylacetate**
   A solution of ethyl 2-aminophenylacetate (5.60g, 0.031 mole) and diisopropylethylamine (7.08ml, 0.042 mole) in dry THF (75ml) was treated with 5 chlorovaleryl chloride (4.00ml, 0.031 mole) and left to stir for 1 h. The reaction mixture was concentrated *in vacuo* and the residue dissolved in ethyl acetate (200ml) and washed with 1M HCI (100ml), dried (Na₂SO₄) and concentrated *in vacuo* to afford a beige solid. This was washed with n-pentane/ether (1:1) and dried to afford the title compound as a light beige solid (8.1g, 91%).
   ¹H NMR (CDCl₃)
   δ: 8.90 (br.s, 1H), 7.88 (d, 1H), 7.05-7.37 (m, 3H), 4.17 (q, 2H), 3.60(s, 2H), 3.45-3.65 (m, 2H) 2.35-2.55 (m, 2H), 1.68-1.98 (m, 4H), 1.28 (t, 3H).
**c) Ethyl 6,7,8,9-tetrahydropyrido[1,2-a]indole-10-carboxylate**
   A solution of ethyl 2-(5-chlorovalerylamino)phenylacetate (8.10g, 0.027 mole) in dry THF (50ml) was added to a stirred suspension of potassium t-butoxide (7.62g, 0.068 mole) in dry THF (200ml) at room temperature under nitrogen. After 1h the purple solution produced was treated with water (10ml) and concentrated *in vacuo.* The residue was shaken with ethyl acetate (200ml) and sat.ammonium chloride solution (150ml), then the organic layer separated, dried (Na₂SO₄) and concentrated *in vacuo* to afford an orange oil. This was chromatographed on silica gel eluting with ether to afford the title compound as a yellow solid (1.25g, 20%).
   ¹H NMR (CDCl₃)
   δ: 8.07-8.17 (m, 1H), 7.13-7.30 (m, 3H), 4.38 (q, 2H), 4.00 (t, 2H), 3.30 (t,2H), 1.82-2.12 (m, 4H), 1.43 (t, 3H).
**d) 6,7,8,9-Tetrahydropyrido[1,2-a]indole-10-carboxylic acid**
   A solution of ethyl 6,7,8,9-tetrahydro-1H-pyrido[1,2-a]indole-10-carboxylate (1.20g, 0.0047 mole) in ethanol (50ml) and 10% NaOH solution (50ml) was heated under reflux for 4 hours. The reaction was then acidified with 1M HCI acid (50ml) and extracted with ethyl acetate (50ml). The organic layer was separated and extracted with 10% Na₂CO₃ solution (120ml) and the aqueous solution then re-acidified with 5M HCI acid and extracted into ethyl acetate (2x75ml). The organic extracts were combined, dried (Na₂SO₄) and concentrated *in vacuo to* afford the title compound (D3) as a white solid (400mg, 40%).
   ¹H NMR (CDCl₃)
   δ: 8.23 (d, 1H), 7.20-7.35 (m, 3H), 4.10 (t, 2H), 3.40 (t, 2H), 2.00-2.15 (m, 2H) 1.85-2.00 (m, 2H).

### Description 4 (intermediate for Examples 11 and 13)

**a) Ethyl 2-(4-chlorobutyrylamino)phenylacetate**
   The title compound was prepared from ethyl 2-aminophenylacetate using the method of Description 3b,and was isolated as a beige solid, (100%).
   ¹H NMR (CDCl₃)
   δ: 8.90 (br,s, 1H), 7.85 (d, 1H), 7.05-7.35 (m, 3H), 4.15(q, 2H), 3.68 (t,2H), 3.60(s, 2H), 2.60 (t, 2H), 2.10-2.30 (m, 2H), 1.26 (t, 3H).
b) **Ethyl 2,3-dihydro-1H-pyrrolo[1,2-a]indole-9-carboxylate**
   The title compound was prepared from ethyl 2-(4-chlorobutyrylamino)phenylacetate using the method of Description 3c, and was isolated as an orange oil that crystallized on standing (15%).
   ¹H NMR (CDCl₃)
   δ: 8.05-8.15 (m, 1H), 7.15-7.30 (m, 3H), 4.35 (q, 2H), 4.06 (t, 2H), 3.28 (t, 2H), 2.55-2.72 (m, 2H), 1.40 (t, 3H).
**c) 2,3-Dihydro-1H-pyrrolo[1,2-a]indole-9-carboxylic acid**
   The title compound (D4) was prepared from ethyl 2,3-dihydro-1H-pyrrolo[1,2-a]indole-9-carboxylate using the method of Description 3d, and was isolated as as an off white solid (42%).
   ¹H NMR (d⁶DMSO)
   δ: 11.85 (br.s, 1H), 7.90-8.02 (m, 1H), 7.32-7.47 (m, 1H), 7.10-7.25 (m, 2H), 4.15 (t, 2H), 3.20(t, 2H), 2.50-2.70 (m, 2H).

### Description 5 (intermediate for Example 12)

a) **Ethyl 2-(6-chlorohexanoylamino)phenylacetate**
   The title compound was prepared from ethyl 2-aminophenylacetate and 6-bromohexanoyl chloride using the method of Description 3b and was isolated as a beige solid (100%).
   ¹H NMR (CDCl₃)
   δ: 8.90(br s, 1H), 7.90(d,1H), 7.05-7.35(m,3H), 4.17(q,2H), 3.60(s,2H), 3.42(t,2H), 2.45(t,2H), 1.45-2.00(m,6H), 1.28(t,3H).
**b) Ethyl 7,8,9,10-tetrahydro-6H-azepino[1,2-a]indole-11-carboxylate**
   The title compound was prepared from ethyl 2-(6-chlorohexanoylamino)phenylacetate using the method of Description 3c, and was purified by chromatography on silica gel eluting with 60-80 petrol/ether (9:1) to afford a white solid (16%).
   ¹H NMR (CDCl₃)
   δ: 8.07-8.19(m,1H), 7.15-7.35(m,3H), 4.40(q,2H), 4.15-4.25(m,2H), 3.45-3.60(m,2H), 1.67-2.00(m,6H), 1.45(t,3H).
**c) 7,8,9,10-Tetrahydro-6H-azepino[1,2-a]indole-11-carboxylic acid**
   The title compound (D5) was prepared from ethyl 7,8,9,10-tetrahydro-6H-azepino[1,2-a]indole-11-carboxylate by hydrolysis with sodium hydroxide as in the method of Description 3d. After 4 hours heating under reflux, the mixture was acidified with 5M HCI acid and the white solid formed filtered off and dried (82%).
   ¹H NMR (d⁶DMSO)
   δ: 12.05(s,1H), 7.94-8.04(m,1H), 7.48-7.60(m,1H), 7.05-7.20(m,2H), 4.24-4.36(m,2H), 3.38-3.53(m,2H), 1.54-1.90(m,6H)

### Description 6 (intermediate for Examples 1, 10, 27 and 29)

### (1-ⁿ-Butyl-4-piperidinyl)methanol

A mixture of ethyl isonipecotate (102g, 0.65 mole) and 1-bromobutane (72 ml, 0.67 mole) in ethanol (1.2L) was treated with anhydrous potassium carbonate (180g, 1.3 mole) and heated under reflux for 2h. The mixture was allowed to cool and then filtered through kieselguhr. The filtrate was concentrated *in vacuo* to leave a yellow oil, which was dissolved in ether (300 ml) and added dropwise over 20 minutes to a stirred suspension of lithium aluminium hydride (50g, 1.3 mole) in either (500 ml) at 0°C under nitrogen. The mixture was stirred at room temperature for 18h, then cooled to 0°C and treated with water (50 ml), 10% NaOH solution (50ml) and water (150ml). The mixture was filtered through keiselguhr and the filtrate concentrated under vacuum to leave a pale yellow oil, which was distilled to afford the title compound as a colourless oil (88.5g, 80%) bp 102-108°C at 0.1 mm Hg.
¹H NMR (CDCl₃)
δ: 3.48(d,2H), 2.88-3.03(m,2H), 2.25-2.38(m,2H), 2.10(br s, 1H), 1.66-2.00(m,4H), 1.17-1.60(m,7H), 0.90(t,3H)

### Description 7 (intermediate for Example 15)

### (1-Benzyl-4-piperidyl)methanol

Ethyl isonipectoate was intially alkylated with benzyl bromide and the product reduced with lithium aluminium hydride using the method of Description 6, to afford the title compound (D7) as a colourless oil (100%).
¹H NMR (CDCl₃)
δ: 7.20-7.35(m,5H), 3.52(s,2H), 3.48(d,2H), 2.86-3.00(m,2H), 1.20-2.05(m,8H).

### Description 8 (intermediate for Examples 13 and 17)

### 6,7-Dihydropyrido[1,2-a]indole-10-carboxylic acid

A stirred solution of methyl 6,7-dihydropyrido[1,2-a]indole-10-carboxylate (T. Teitei and LK. Dalton, Australian J. Chem 1969, 22, 997) (1.0g, 0.0044 mole) in methanol (40ml) was treated with a solution of potassium hydroxide (3.0g, 0.054 mole) in water (50ml) and heated under reflux for 3 h. The solution was allowed to cool, then acidified with HCI acid and extracted with ethyl acetate. The extract was dried (Na₂SO₄) and concentrated under vacuum to leave the title compound (D8) as a yellow solid (600mg, 64%).
¹H NMR (CDCl₃)
δ: 8.18-8.22 (m, 1H), 7.50 (d, 1H), 7.20-7.35 (m,3H), 6.27-6.38 (m, 1H), 4.15 (t, 2H), 2.62-2.78 (m, 2H).

### Description 9 (intermediate for Examples 18)

### Pyrido[1,2-a]indole-10-carboxylic acid

The title compound (D9) was prepared from methyl pyrido[1,2-a]indole-10-carboxylate (T. Teitei and L.K. Dalton, Australian J. Chem. 1969, 22, 997) using the method of Description 8 as a bright yellow solid (76%).
¹H NMR (CDCl₃ + CD₃OD)
δ: 8.56(d, 1H), 8.34-8.46 (m, 2H), 7.93 (d,1H), 7.32-7.57 (m, 3H), 6.87 (t, 1H).

### Description 10 (intermediate for Example 20)

### (1-Benzyl-4-piperidyl)methylamine(D10)

Isonipecotamide was initially alkylated with benzyl bromide, then the amide dehydrated with phosphorus pentoxide and the resulting nitrile reduced with lithium aluminium hydride using the method of Description 1a to afford the title compound as a colourless oil after distillation (67%) bp 106°C at 0.25 mmHg.
¹H NMR (CDCl₃)
δ: 7.20-7.37(m,5H), 3.48(s,2H), 2.85-2.95(m,2H), 2.55(d,2H), 1.87-2.00(m,2H), 1.60-1.75(m,2H), 1.10-1.40(m,5H).

### Description 11 (intermediate for Example 29)

a) Methyl 2-chloroindole-3-carboxylate
   A stirred suspension of methyl indole-3-carboxylate (6.0g, 0.034 mole) in chloroform (200 ml) was treated with N-chlorosuccinimide (5.04g, 0.038 mole) to afford a clear solution within 15 minutes. After 2h at room temperature this was treated with 1M HCl/ether (34 ml, 0.034 mole) and allowed to stir for a further 1h, then treated with excess 10% Na₂CO₃ solution and the chloroform layer separated, dried (Na₂SO₄) and concentrated *in vacuo.* The residual yellow solid was recrystallised from chloroform/60-80 petrol to afford the title compound (D11a) as a beige solid (3.4g, 48%).
   ¹H NMR (CDCl₃/d⁶DMSO)
   δ: 11.3(br s,1H), 8.02-8.12(m,1 H), 7.30-7.40(m,1 H), 7.1 8-7.26(m,2H), 3.95(s,3H).
   MS (El) M⁺ 209 and 211
**b) Methyl 1-methyl-1,2,3,4-tetrahydropyrimido[1,2-a]indole-10**-carboxylate
   A solution of methyl 2-chloroindole-3-carboxylate (3.4g, 0.016 mole) in dry THF (70 ml) at 5°C under nitrogen was treated portionwise with sodium hydride (480mg of 80% oil dispersion, 0.016 mole) and then stirred at room temperature for 30 mins. The resulting solution was treated with a solution of 3,3-dimethylaminopropyl chloride (0.020 mole) in toluene (30 ml) and heated under reflux for 48h, then concentrated *in vacuo* and the residue treated with 10% Na₂CO₃ solution (50 ml) and extracted with ethyl acetate (2 x 70 ml). The combined extracts were dried (Na₂SO₄) and concentrated *in vacuo* to leave a yellow oil, which was chromatographed on silica gel eluting with ether/60-80 petrol (1:1). The title compound (D11) was obtained as a beige solid (1.95g, 50%).
   ¹H NMR (CDCl₃)
   δ: 7.92(d,1H), 6.97-7.19(m,3H), 3.92(t,2H), 3.88(s,3H), 3.36(t,2H), 3.27(s,3H), 2.10-2.22(m,2H).

### Description 12 (intermediate for Example 26)

### eq-Quinolizidin-2-ylmethylamine

A stirred suspension of lithium aluminium hydride (400mg, 0.010 mole) in THF (20 ml) at room temperature under nitrogen was treated with a solution of *eq*-2-cyanoquinolizidine (E. Koshinaka et al, Yakugaku Zasshi 1980, 100, 88) in THF (3ml) and the mixture then heated under reflux for 20 minutes. The mixture was allowed to cool then treated cautiously with water (0.4 ml), 10% NaOH solution (0.4 ml) and water (1.2 ml). The resulting mixture was filtered and the filtrate concentrated *in vacuo.* The residue was distilled in a Kugelrohr apparatus to afford the title compound (D13) as a colourless oil (700mg, 97%).
¹H NMR (CDCl₃)
δ: 2.80-2.92(m,2H), 2.57(d,2H), 1.94-2.12(m,2H), 1.20-1.80(m,13H), 0.88-1.05(m,1H).

### Description 13 (intermediate for Example 30)

### 3-Methylthiazolo[3,2-a]indole-9-carboxylic acid

a) A stirred solution of 3-methylthiazolo[3,2-a]indole (A. Kiprianov and V. Khilya, Zh. Organ. Khim. 1966, 2, 1474) (270mg, 0.0014 mole) in DMF (3 ml) was cooled to 5°C under argon and treated with triflouroacetic anhydride (0.23 ml, 0.0017 mole), then allowed to warm to room temperature over 3h. The solution was poured into water (25 ml) and the mixture extracted with ethyl acetate (2 x 20 ml). The combined extracts were dried (Na₂SO₄) and then concentrated *in vacuo* to afford 3-methyl-9-triflouroacetylthiazolo[3,2-a]indole (370mg, 90%) as a brown solid.
   ¹H NMR (CDCl₃)
   δ: 8.10(br s,1H),7.85(d,1H), 7.39-7.47(m,1H), 7.25-7.35(m,1H), 6.69(s,1H), 2.83(s,3H).
b) 3-Methyl-9-trifluoroacetylthiazolo[3,2-a]indole (370mg, 0.0013 mole) was treated with 20% NaOH solution (15 ml) and ethanol (15 ml) and heated under reflux for 6h. The mixture was concentrated *in vacuo* to half its volume and the residue acidified with 2M HCI acid and then extracted with ethyl acetate (2 x 30 ml). The combined extracts were dried (Na₂SO₄) and concentrated *in vacuo* to afford the title compound (D13) as a brown solid (300mg, 100%).
   ¹H NMR (d⁶DMSO)
   δ: 12.3(v br s, 1H),7.93-8.08(m,2H), 7.16-7.40(m,2H), 6.95(s,1H), 2.59(s,3H).

### Description 14 (intermediate for Example 25)

### N-(2-Bromoethyl)methanesulphonamide

A stirred solution of 2-bromoethylamine hydrobromide (5.10g, 0.025 mole) and triethylamine (6.96g, 0.050 mole) in dichloromethane (200 ml) at ice bath temperature was treated dropwise with methanesulphonyl chloride (1.96 ml, 0.025 mole). The mixture was allowed to warm to room temperature and stir for 16h, then washed with water and 5M HCI acid, dried (Na₂SO₄) and concentrated *in vacuo* to afford the title compound (D14) as a colourless oil which solidified on standing to give a white solid (3.5g, 69%).
¹H NMR (CDCl₃)
δ: 4.92(s,1H), 3.62-3.48(m,4H), 3.05(s,3H).

### Description 15 (intermediate for Example 31)

### a) 2,3-Dihydrothiazolo[3,2-a]indole

A solution of thioxindole (400mg, 0.0027 mole) and 1,2-dibromoethane (0.24 ml, 0.0027 mole) in dry THF (10 ml) was added to a stirred solution of potassium t-butoxide (760mg, 0.0068 mole) in dry THF (40 ml) at room temperature under argon. The mixture was stirred for 3h, then treated with water (100 ml) and extracted with ethyl acetate (2 x 70 ml). The combined extracts were dried (Na₂SO₄) and concentrated *in vacuo* to leave an orange oil, which was chromatographed on silica gel eluting with 10% ether/60-80 petrol. The title compound was obtained as a white solid (135mg, 29%).
¹H NMR (CDCl₃)
δ: 7.42-7.23(m,1H), 7.00-7.25(m,3H), 6.20(s,1H), 4.23(t,2H), 3.79(t,2H).

### b) 2,3-Dihydrothiazolo[3,2-a]indole-9-carboxylic acid

2,3-Dihydrothiazolo[3,2-a]indole was treated with trifluoroacetic anhydride using the method of Description 13a to afford 9-trifluoroacetylthiazolo[3,2-a]indoleas a purple solid (85%).
¹H NMR (CDCl₃)
δ: 7.93(br s, 1H), 7.07-7.30(3H), 4.30(t,2H), 3.85(t,2H).

The title compound (D15b) was prepared from 9-trifluoroacetyl-2,3-dihydrothiazolo[3,2-a]indole using the method of Description 13b to give a purple solid (95%), which was used without purification.

### Description 16

a) **Thiazolo[3,2-a]indole**
   A stirred solution of thioxindole (3.8g, 0.025 mole) and bromoacetaldehyde diethyl acetal (3.9 ml, 0.026 mole) in acetone (200 ml) was treated with anhydrous potassium carbonate (6.9g, 0.050 mole) and the mixture heated under reflux for 2h followed by 12h at room temperature. The mixture was concentrated *in vacuo* and the residue treated with water (100 ml) and extracted with ethyl acetate (2 x 100 ml). The combined extracts were dried (Na₂SO₄), concentrated *in vacuo* and the residue chromatographed on silica gel eluting with 10% ether/60-80 petrol to afford 2-(2,2-diethoxyethylmercapto)indole (3.0g, 44%) as a yellow oil.
   ¹H NMR (CDCl₃)
   δ: 9.30(br s,1H), 7.52(d,1H), 7.28(d,1H), 7.04-7.20(m,2H), 6.58(s,1H), 4.72(t,1H), 3.55-3.85(m,4H), 3.05(d,2H), 1.31(t,6H).
   A well stirred mixture of 2-(2,2-diethoxyethylmercapto)indole (1.5g, 0.0057 mole) in polyphosphoric acid (30g) was heated to 130°C for 20 minutes, then allowed to cool to room temperature and the mixture diluted with water (300 ml). The resulting aqueous solution was basified by addition of solid potassium carbonate and then extracted with ethyl acetate (2 x 120 ml). The combined extracts were dried (Na₂SO₄), concentrated *in vacuo* and the residue chromatographed on silica gel eluting with 10% ether/60-80 petrol to afford the title compound as a white solid (0.56g, 57%).
   ¹H NMR (CDCl₃)
   δ: 7.60-7.70(m,3H), 7.11-7.28(m,2H), 6.60(d,1H), 6.53(s,1H).
**b) Thiazolo[3,2-a]indole-9-carboxylic acid**
   "Thiazolo[3,2-a]indole was treated with trifluoroacetic anhydride using the method of Description 13a to afford 9-trifluoroacetylthiazolo[3,2-a]indole as a beige solid (95%).
   ¹H NMR (CDCl₃)
   δ: 8.06(br s,1H), 7.94(d,1H), 7.69(d,1H), 7.39-7.48(m,1H), 7.30-7.37(m,1H), 7.18(d,1H).
   The title compound (D16b) was prepared from
   9-trifluoroacetylthiazolo[3,2-a]indole using the method of Description 13b and was isolated as a light purple solid (84%).
   ¹H NMR (CDCl₃/d⁶DMSO)
   δ: 7.98-8.08(m,2H), 7.73(d,1H), 7.10-7.31(m,2H), 7.00(d,1H).

### Description 17

### Methyl 2,4-dimethylpyrimido[1,2-a]indole-10-carboxylate

A stirred solution of methyl 2-aminoindole-3-carboxylate (I. Forbes et al, J. Chem. Soc. Perkin I, 1992, 275) (0.25g, 0.0013 mole) in xylene (5 ml) was treated with 2,4-pentanedione (0.13g, 0.0013 mole) and a few crystals of 4-toluenesulphonic acid and heated under reflux for 2h. The mixture was concentrated *in vacuo* and the residue dissolved in chloroform (20 ml), washed with water (2 x 20 ml), dried (MgSO₄) and concentrated in *vacuo* to afford the title compound as a brown solid (0.25g, 75%).
¹H NMR (CDCl₃)
δ: 8.58(d,1H), 8.09(d,1H), 7.52(dt,1H), 7.34(dt,1H), 6.53(s,1H), 4.06(s,3H), 3.03(s,3H), 2.68(s,3H).

### Description 18

### Methyl 1,2,3,4-tetrahydropyrimido[1,2-a]indole-10-carboxylate

A solution of methyl 2-chloroindole-3-carboxylate (D11a, 1.5g, 0.0071 mole) in THF (30 ml) under argon was treated with sodium hydride (215mg of 80% oil dispersion, 0.0071 mole) and stirred for 20 minutes. The resulting solution was treated with a solution of 3-bromopropylamine (0.0093 mole) in toluene (15 ml) and a white gelatinous precipitate formed. This mixture was diluted with more THF (30 ml) and heated under reflux for 18h, then concentrated *in vacuo* and the residue shaken well with ethyl acetate (40 ml) and 10% Na₂CO₃ solution (30 ml). The organic layer was separated, dried (Na₂SO₄) and concentrated *in vacuo* to leave a beige solid. This was chromatographed on silica gel eluting with ether/60-80 petrol (1:1) to afford unreacted starting material (600mg) and the title compound (D18) as a white solid (110mg, 6%).
¹H NMR (d⁶DMSO)
δ: 7.58(d,1H), 7.26(br s,1H), 7.12(d,l H), 6.88-7.05(m,2H), 3.98(t,2H), 3.73(s,3H), 3.38-3.46(m,2H), 2.08(quintet,2H).

### Description 19

### Methyl pyrimido[1,2-a]indole-10-carboxylate

A stirred solution of methyl 2-aminoindole-3-carboxylate (I. Forbes et al, J. Chem. Soc. Perkin I, 1992, 275) (0.5g, 0.0026 mole) in xylene (10 ml) was treated with 1,1,3,3-tetramethoxypropane (0.43g, 0.0026 mole) and a few crystals of 4-toluenesulphonic acid and heated under reflux for 2.5h. The mixture was concentrated *in vacuo* and the residue dissolved in chloroform (25 ml), washed with water (2 x 10 ml), dried (MgSO₄) and concentrated *in vacuo* to leave a dark orange solid. This was purified by chromatography on silica gel eluting with ethyl acetate to afford the title compound (D19) as an orange solid (0.23g, 35%).
¹H NMR (CDCl₃)
δ: 8.68-8.78(m,2H), 8.57(d,1H), 7.89(d,1H), 7.59(dt,1H), 7.45(dt,1H), 6.80-6.90(m,1H), 4.08(s,3H).

### 5-HT₄ RECEPTOR ANTAGONIST ACTIVITY

### 1) Guinea pig colon

Male guinea-pigs, weighing 250-400g are used. Longitudinal muscle-myenteric plexus preparations, approximately 3cm long, are obtained from the distal colon region. These are suspended under a 0.5g load in isolated tissue baths containing Krebs solution bubbled with 5% CO₂ in O₂ and maintained at 37°C. In all experiments, the Krebs solution also contains methiothepin 10⁻⁷M and granisetron 10⁻⁶M to block effects at 5-HT₁, 5-HT₂ and 5-HT₃ receptors.

After construction of a simple concentration-response curve with 5-HT, using 30s contact times and a 15min dosing cycle, a concentration of 5-HT is selected so as to obtain a contraction of the muscle approximately 40-70% maximum(10⁻⁹M approx). The tissue is then alternately dosed every 15min with this concentration of 5-HT and then with an approximately equi-effective concentration of the nicotine receptor stimulant, dimethylphenylpiperazinium (DMPP). After obtaining consistent responses to both 5-HT and DMPP, increasing concentrations of a putative 5-HT₄ receptor antagonist are then added to the bathing solution. The effects of this compound are then determined as a percentage reduction of the contractions evoked by 5-HT or by DMPP. From this data, plC₅₀ values are determined, being defined as the -log concentration of antagonist which reduces the contraction by 50%. A compound which reduces the response to 5-HT but not to DMPP is believed to act as a 5-HT₄ receptor antagonist.

Compounds were generally active in the range of concentrations of the order of plC₅₀=7 or more, E1, E2, E4, E6, E8, E15 and E27 showing particularly good activity when Y is 0, and E3, E20, E23 and E28 showing particularly good activity when Y is NH.

### 2) Piglet Atria

Compounds are tested in the piglet spontaneous beating screen (Naunyn-Schmiedeberg's Arch. Pharmacol 342, 619-622). pK_{B} (-log₁₀ K_{B}) value for the compound of Example 3 was 10.05.

### 3) Rat oesophagus

Rat oesophageal tunica muscularis mucosae is set up according to Baxter *et. al.* Naunyn-Schmiedeberg's Arch. Pharmacol., 343, 439-446 (1991). The inner smooth muscle tube of the muscularis mucosae is isolated and mounted for isometric tension recording in oxygenated (95% O₂/5% CO₂) Tyrodes solution at 37°C. All experiments are performed in pargyline pre-treated preparations (100µM for 15 min followed by washout) and in the presence of cocaine (30µM). Relaxant responses to 5-HT are obtained after pre-contracting the oesophagus tissue with carbachol (3µM).

### 4) 5-HT-induced motility in dog gastric pouch

Compounds are tested for inhibition in the *in vivo* method described in "Stimulation of canine motility by BRL 24924, a new gastric prokinetic agent", Bermudez *et al,* J. Gastrointestinal Motility, 1990, 2(4), 281-286.

### IN VIVO TESTING FOR ANXIOLYTIC ACTIVITY

### Social Interaction Test in Rats

Rats (male, Sprague Dawleys, Charles River, 250-300g) are housed in groups of eight in a holding room for 5 days. They are then housed singly in a room adjacent to the experimental room for 4 days prior to the experimental day. On the experimental day rats are administered vehicle, test compound or a benzodiazepine anxiolytic, chlordiazepoxide, p.o. in pairs (n=8-16), at 15 minute intervals beginning at 10.00 a.m. 30 mins. later they are placed with a weight matched pair-mate (encountered for the first time) in the social interaction box in a separate room. The box is made of white perspex 54 cm x 37 cm x 26 cm with a transparent perspex front side and no lid. The floor is divided up into 24 squares and the box is brightly lit (115 lux). Active social interactive behaviours (grooming, sniffing, climbing over or under, following, biting, mounting and boxing) are scored blind for the next 15 min by remote video monitoring to give total interaction scores. The number of squares crossed by each rat is also scored and summed. After the end of each test the box is carefully wiped.

E3 increased total interaction scores over the dose range 0.01 - 10 mg/kg p.o.

## Claims

1. A compound of formula (I), or a pharmaceutically acceptable salt thereof: 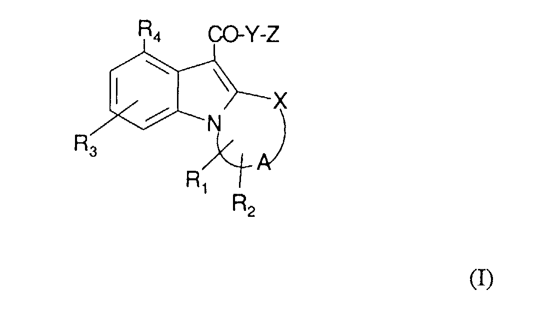 wherein
X is O, S, SO, SO₂, CH₂, CH or NR wherein R is hydrogen, C₁₋₆ alkyl or C₃₋₆ cycloalkyl;
A is a saturated or unsaturated polymethylene chain of 2 - 4 carbon atoms;
R₁ and R₂ are hydrogen, C₁₋₆ alkyl or C₃₋₆ cycloalkyl;
R₃ is hydrogen, halo, C₁₋₆ alkyl, C₃₋₆ cycloalkyl, amino, nitro or C₁₋₆ alkoxy;
R₄ is hydrogen, halo, C₁₋₆ alkyl, C₃₋₆ cycloalkyl or C₁₋₆ alkoxy;
Y is O or NH;
Z is of sub-formula (a), (b) or (c): 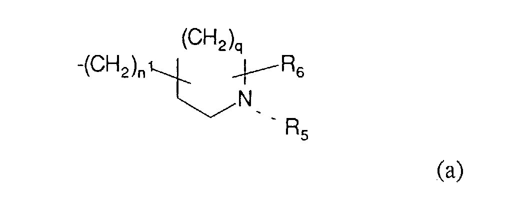 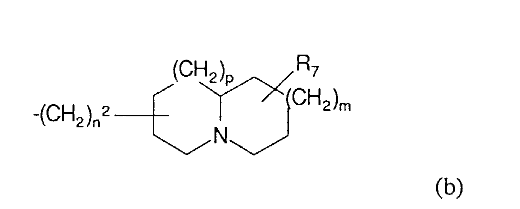 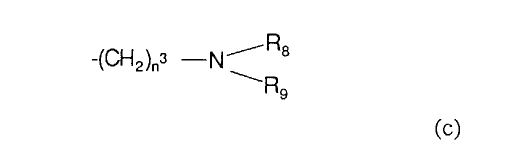 wherein
n¹ is 1, 2, 3 or 4; n² is 0, 1, 2, 3 or 4; n³ is 2, 3, 4 or 5;
q is 0, 1, 2 or 3; p is 0, 1 or 2; m is 0, 1 or 2;
R₅ is hydrogen, C₁₋₁₂ alkyl, aralkyl or R₅ is (CH₂)_{z}-R₁₀ wherein z is 2 or 3 and
R₁₀ is selected from cyano, hydroxyl, C₁₋₆ alkoxy, phenoxy, C(O)C₁₋₆ alkyl, COC₆H₅, -CONR₁₁R₁₂, NR₁₁COR₁₂, SO₂NR₁₁R₁₂ or NR₁₁SO₂R₁₂
wherein R₁₁ and R₁₂ are hydrogen, C₁₋₆ alkyl or C₃₋₆ cycloalkyl;
R₆, R₇ and R₈ are independently hydrogen, C₁₋₆ alkyl or C₃₋₆ cycloalkyl ;
R₉ is hydrogen, C₁₋₆ alkyl or C₃₋₆ cycloalkyl; and
the dotted line in sub-formula (a) indicates the R₅ group is absent when the -(CH₂)ₙ1- group is directly attached to the nitrogen of the azacycle;
or a compound of formula (I) wherein the CO-Y linkage is replaced by a heterocyclic bioisostere of formula (d): 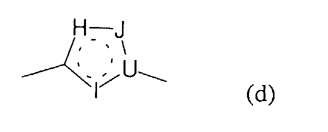 wherein
the dotted circle represents one or two double bonds in any position in the 5-membered ring; H, J and I independently represent oxygen, sulphur, nitrogen or carbon, provided that at least one of H, J and I is other than carbon; U represents nitrogen or carbon;
other than the compound of formula (I) in which X is O, A is -(CH₂)₃-, R₁ R₂ R₃ and R₄ are hydrogen, Y is NH, Z is of sub-formula (a) in which n¹ is 1,
q is 3 , R₅ is n-butyl and R₆ is hydrogen;
having 5-HT₄ receptor antagonist activity.

2. A compound according to claim 1 wherein X is O.

3. A compound according to claim 1 or 2 wherein A is -(CH₂)₃-.

4. A compound according to claim 1, 2 or 3 wherein R₁ and R₂ are independently hydrogen or methyl.

5. A compound according to claim 1, 2, 3 or 4 wherein R₃ is hydrogen and R₄ is hydrogen or halo.

6. A compound according to any one of claims 1 to 5 wherein Y is O or NH.

7. A compound according to any one of claims 1 to 6 wherein Z is of sub-formula (a) and (CH₂)ₙ1 is attached at a carbon atom of the azacycle.

8. A compound according to claim 7 wherein Z is N-substituted 4-piperidylmethyl.

9. A compound according to claim 8 wherein the N-substituent is C₂ or greater alkyl, or optionally substituted benzyl.

10. A compound according to claim 1 selected from the compounds E1 to E46 inclusive (other than compound E3), as defined herein and where A is a group of formula -CH₂-(CH₂)ᵣ-CH₂-, including pharmaceutically acceptable salts thereof
| | **R**_{**1**} | **R**_{**2**} | **r** | **R**_{**3**} | **R**_{**4**} | **X** | Y | Z |
|---|---|---|---|---|---|---|---|---|
| **E1** | H | H | 1 | H | H | O | O | (i) |
| **E2** | H | H | 1 | H | H | O | O | (vi) |
| **E3** | H | H | 1 | H | H | O | NH | (i) |
| **E4** | H | H | 1 | H | H | O | O | (iii) |
| **E5** | H | H | 1 | H | H | O | NH | (iii) |
| **E6** | H | H | O | H | H | O | O | (i) |
| **E7** | 3-(CH₃)₂ | | 1 | H | H | O | O | (i) |
| **E8** | H | H | 1 | H | H | S | O | (i) |
| **E9** | H | H | 2 | H | H | O | O | (i) |
| **E10** | H | H | 1 | H | H | CH₂ | O | (i) |
| **E11** | H | H | O | H | H | CH₂ | O | (i) |
| **E12** | H | H | 2 | H | H | CH₂ | O | (i) |
| **E13** | H | H | O | H | H | CH₂ | NH | (i) |
| **E14** | H | H | O | H | H | O | NH | (i) |
| **E15** | H | H | 1 | H | H | O | O | Bzppm |
| **E16** | H | H | 1 | H | H | SO | O | (i) |
| **E17** | -- | Δ | - | H | H | CH | O | (i) |
| **E18** | -- | τ | - | H | H | CH | O | (i) |
| **E19** | H | H | 1 | H | H | S | NH | (i) |
| **E20** | H | H | 1 | H | H | O | NH | Bzppm |
| **E21** | H | H | 1 | H | H | O | NH | ppm |
| **E22** | H | H | 1 | H | H | O | NH | ⁿC₆H₁₃ppm |
| **E23** | H | H | 1 | H | H | O | NH | (ii) |
| **E24** | H | H | 1 | H | H | O | NH | Etppm |
| **E25** | H | H | 1 | H | H | O | NH | MeSO₂aEtppm |
| **E26** | H | H | 1 | H | H | O | NH | (vi) |
| **E27** | H | H | 1 | 8-F | H | O | O | (i) |
| **E28** | H | H | 1 | 8-F | H | O | NH | (i) |
| **E29** | H | H | 1 | H | H | NMe | O | (i) |
| **E30** | -- | π | -- | H | H | S | O | (i) |
| **E31** | H | H | O | H | H | S | O | (i) |
| **E32** | -- | ϑ | - | H | H | S | O | (i) |
| **E33** | -- | Λ | - | H | H | N | O | (i) |
| **E34** | H | H | O | H | H | S | NH | (i) |
| **E35** | -- | ϑ | -- | H | H | S | NH | (i) |
| **E36** | H | H | 1 | H | H | NH | O | (i) |
| **E37** | H | H | O | H | H | O | O | (vi) |
| **E38** | H | H | 2 | H | H | O | NH | (i) |
| **E39** | - | τ | - | H | H | N | O | (i) |
| **E40** | H | H | O | H | H | S | O | (vi) |
| **E41** | H | H | O | H | H | S | NH | (vi) |
| **E42** | -- | ϑ | -- | H | H | S | O | (vi) |
| **E43** | -- | ϑ | -- | H | H | S | NH | (vi) |
| **E44** | H | H | 1 | H | H | S | O | (vi) |
| **E45** | -- | τ | -- | H | H | NH | NH | (i) |
| **E46** | H | H | 1 | H | H | N | NH | (i) |
wherein the variables are defined as follows:- 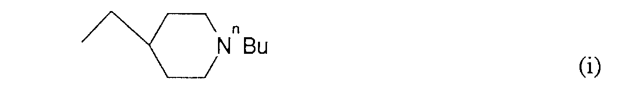 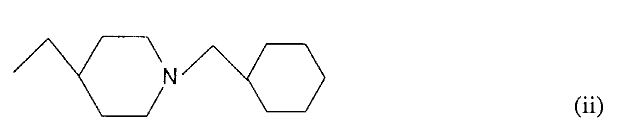 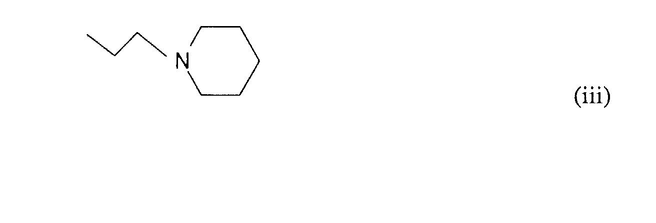 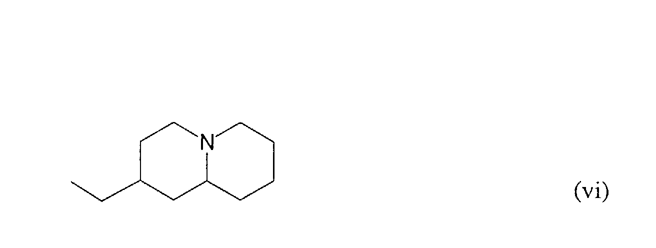
Δ - AR₁R₂ is -(CH₂)₂-CH= Bz - benzyl
Γ - AR₁R₂ is -CH=CH-CH= ppm - 4-piperidylmethyl
π - AR₁R₂ is -C(CH₃)=CH- aEt - aminoethyl
ϑ - AR₁R₂ is -CH=CH-
Δ - AR₁R₂ is -C(CH₃)=CH-C(CH₃)=

11. A process for preparing the ester or amide compounds according to claim 6, which comprises reacting an appropriate indole 10-carboxylic acid derivative with an appropriate alcohol or amine.

12. A pharmaceutical composition comprising a compound according to any one of claims 1 to 10, and a pharmaceutically acceptable carrier.

13. A compound according to claim 1 for use as an active therapeutic substance.

14. The use of a compound according to claim 1 in the manufacture of a medicament for use as a 5-HT₄ receptor antagonist.

15. The use according to claim 14 for use as a 5-HT₄ antagonist in the treatment or prophylaxis of gastrointestinal disorders, cardiovascular disorders and CNS disorders.

## Patentansprüche

1. Verbindung der Formel (1) oder ein pharmazeutisch verträglichcs Salz davon: 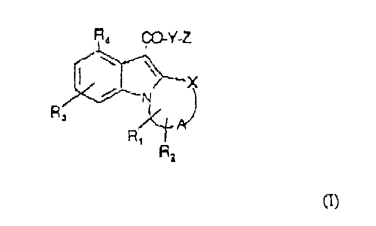 wobei
X für O, S, SO, SO₂, CH₂, CH oder NR steht, wobei R ein Wasserstoffatom, C₁₋₆-Alkyl- oder C₃₋₆-Cycloalkylrest ist;
A für eine gesättigte oder ungesättigte Polymethylenkette mit 2 bis 4 Kohlenstoffatomen steht;
R₁ und R₂ ein Wasserstoffatom, C₁₋₆-Alkyl- oder C₃₋₆-Cycloalkylrest ist;
R₃ ein Wasserstoffatom, Halogenatom, C₁₋₆-Alkyl-, C₃₋₆-Cycloalkyl-, Amino-, Nitro- oder C₁₋₆-Alkoxyrest ist;
R₄ ein Wasserstoffatom, Halogenatom, C₁₋₆Alkyl-, C₃₋₆-Cycloalkyl- oder C₁₋₆-Alkoxy-rest ist;
Y für O oder NH steht;
Z die Unterformel (a), (b) oder (c) ist: 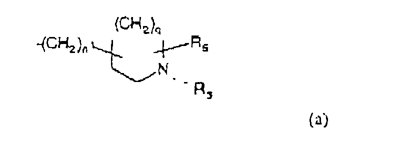 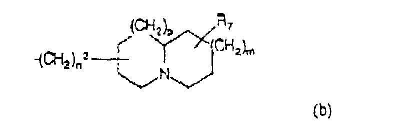 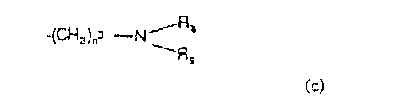 wobei
n¹ für 1, 2, 3 oder 4 steht; n¹ für 0, 1, 2, 3 oder 4 steht; n³ für 2, 3, 4 oder 5 steht;
q für 0, 1, 2 oder 3 steht; p für 0, 1 oder 2 steht; m für 0, 1 oder 2 steht;
R₅ ein Wasserstoffatom, C₁₋₁₂-Alkylrest, Aralkylrest oder R₅ (CH₂)_{z}-R₁₀ ist, wobei z für 2 oder 3 steht und
R₁₀ gewählt wird aus den Resten Cyano, Hydroxyl, C₁₋₆-Alkoxy, Phenoxy, C(O)C₁₋₆-Alkyl, COC₆H₅, -CONR₁₁R₁₂, NR₁₁COR₁₂, SO₂NR₁₁R₁₂ oder NR₁₁SO₂R₁₂,
wobei R₁₁ und R₁₂ ein Wasserstoffatom, C₁₋₆-Alkyl- oder C₃₋₆-Cycloalkyl-rest ist;
R₆, R₇ und R₈ unabhangig voneinander ein Wasserstoffatom, C₁₋₆-Alkyl- oder C₃₋₆-Cycloalkylrest sind;
R₉ ein Wasserstoffatom, C₁₋₆-Alkyl- oder C₃₋₆-Cycloalkylrest ist; und
die unterbrochene Linie in Unterformel (a) anzeigt, daß der Rest R₅ fehlt, wenn der Rest -(CH₂)ₙ₁ - direkt an den Stickstoff des Azacyclus gebunden ist;
oder eine Verbindung der Formel (I), wobei die Verknüpfung CO-Y durch ein heterocyclisches Bioisoster der Formel (d) ersetzt ist: 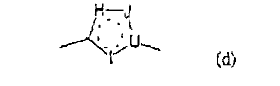 wobei
der unterbrochene Kreis für eine oder zwei Doppelbindungen in beliebiger Position im 5-gliedrigen Ring steht; H, J und I unabhängig voneinander ein Sauerstoff-, Schwefel-, Stickstoff- oder Kohlenstoffatom darstellen, mit der Maßgabe, daß wenigstens eines der Atome H, J und I von Kohlenstoff verschieden ist;
U ein Stickstoff- oder Kohlenstoffatom darstellt;
verschieden von der Verbindung der Formel (I) in der X für O steht, A für -(CH₂)₃- steht, R₁, R₂, R₃ und R₄ Wasserstoffatome sind, Y für NH steht, Z die Unterformel (a) besitzt, bei der n¹ für steht, q für 3 steht, R₅ ein n-Butylrest ist und R₅ ein Wasserstoffatom ist;
die 5-HT₄-Rezeptoräritagonistaktivität besitzen.

2. Verbindung nach Anspruch 1, in der X für O steht.

3. Verbindung nach Anspruch 1 oder 2, in der A für -(CH₂)₃- steht.

4. Verbindung nach Anspruch 1, 2 oder 3, in der R₁ und R₂ unabhängig voneinander ein Wasserstoffatom oder eine Methylgruppe darstellen.

5. Verbindung nach Anspruch 1, 2, 3 oder 4, in der R₃ eiii Wasserstoffatom ist und R₄ ein Wasserstoff- oder halogenatom ist.

6. Verbindung nach einem der Ansprüche 1 bis 5, in der Y für O oder NH steht.

7. Verbindung nach einem der Ansprüche 1 bis 6, in der Z die Unterformel (a) besitzt und (CH₂)ₙ₁ an ein Kohlenstoffatom des Azacyclus gebunden ist.

8. Verbindung nach Anspruch 7, in der Z eine N-substitutierte 4-Piperidylmethylgruppe ist.

9. Verbindung nach Anspruch 8, in der der N-Substituent ein C₂- oder größerer Alkylrest oder eine gegebenenfalls substitutierte Benzylgruppe ist.

10. Verbindung nach Anspruch 1, ausgewählt aus den hier definierten Verbindungen E1 bis einschließlich E46 (außer Verbindung E3) und wobei A ein Rest der Formel -CH₂-(CH₂)₋CH₂- ist, einschließlich pharmazeutisch verträglicher Salze davon,
| | R₁ | R₂ | r | R₃ | R₄ | X | Y | Z |
|---|---|---|---|---|---|---|---|---|
| E1 | H | H | 1 | H | H | O | O | (i) |
| E2 | H | H | 1 | H | H | O | O | (vi) |
| E3 | H | H | 1 | H | H | O | NH | (i) |
| E4 | H | H | 1 | H | H | O | O | (iii) |
| E5 | H | H | 1 | H | H | O | NH | (iii) |
| E6 | H | H | H | H | O | O | O | (i) |
| E7 | 3-(CH₃)₂ | | 1 | H | H | O | O | (i) |
| E8 | H | H | 1 | H | H | S | O | (i) |
| E9 | H | H | 2 | H | H | O | O | (i) |
| E10 | H | H | 1 | H | H | CH₂ | O | (i) |
| E11 | H | H | O | H | H | CH₂ | O | (i) |
| E12 | H | H | 2 | H | H | CH₂ | O | (i) |
| E13 | H | H | O | H | H | CH₂ | NH | (i) |
| E14 | H | H | O | H | H | O | NH | (i) |
| E15 | H | H | 1 | H | H | O | O | Bzppm |
| E16 | H | H | 1 | H | H | SO | O | (i) |
| E17 | - | Δ | - | H | H | CH | O | (i) |
| E18 | - | Γ | | H | H | CH | O | (i) |
| E19 | H | H | 1 | H | H | S | NH | (i) |
| E20 | H | H | 1 | H | H | O | NH | Bzppm |
| E21 | H | H | 1 | H | H | O | NH | ppm |
| E22 | H | H | 1 | H | H | O | NH | ⁿC₆H₁₃ppm |
| E23 | H | H | 1 | H | H | O | NH | (ii) |
| E24 | H | H | 1 H | H | H | O | NH | Etppm |
| E25 | H | H | 1 | H | H | O | NH | MeSO₂aEtppm |
| E26 | H | H | 1 | H | H | O | NH | (vi) |
| E27 | H | H | 1 | 8-F | H | O | O | (i) |
| E28 | H | H | 1 | 8-F | H | O | NH | (i) |
| E29 | H | H | 1 | H | H | NMe | O | (i) |
| E30 | - | π | - | H | H | S | O | (i) |
| E31 | H | H | O | H | H | S | O | (i) |
| E32 | - | ϑ | | H | H | S | O | (i) |
| E33 | - | Λ | Δ | H | H | N | O | (i) |
| E34 | H | H | O | H | H | S | NH | (i) |
| E35 | - | ϑ | - | H | H | S | NH | (i) |
| E36 | H | H | 1 | H | H | NH | O | (i) |
| E37 | H | H | O | H | H | O | O | (vi) |
| E38 | H | H | 2 | H | H | O | NH | (i) |
| E39 | - | τ | - | H | H | N | O | (i) |
| E40 | H | H | O | H | H | S | O | (vi) |
| E41 | H | H | O | H | H | S | NH | (vi) |
| E42 | - | ϑ | - | H | H | S | O | (vi) |
| E43 | - | ϑ | - | H | H | S | NH | (vi) |
| E44 | H | H | 1 | H | H | S | O | (vi) |
| E45 | - | Γ | - | H | H | NH | NH | (i) |
| E46 | H | H | 1 | H | H | N | NH | (i) |
wobei die Variablen wie folgt definiert sind: 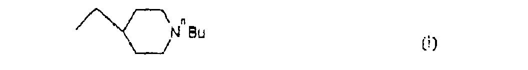 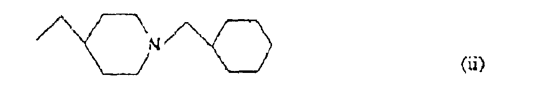 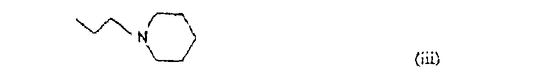 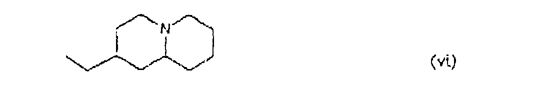
Δ - AR₁R₂ steht für -(CH₂)₂-CH= Bz - Benzyl
Γ - AR₁R₂ steht für -CH=CH-CH= ppm - 4-Piperidylmethyl
π - AR₁R₂ steht für -C(CH₃)=CH- aEt - Aminoethyl
ϑ - AR₁R₂ steht für -CH=CH-
Λ - AR₁R₂ steht für -C(CH₃)=CH-C(CH₃)=

11. Herstellungsverfahren für die Ester- oder Amidverbindungen nach Anspruch 6, umfassend Umsetzen eines geeigneten Indol-10-carbonsäurederivats mit einem geeigneten Alkohol oder Amin.

12. Arzneimittel, umfassend eine Verbindung nach einem der Ansprüche 1 bis 10 sowie einen pharmazeutisch verträglichen Träger.

13. Verbindung nach Anspruch 1 zur Verwendung als wirksames Therapeutikum.

14. Verwendung einer Verbindung nach Anspruch 1 bei der Herstellung eines Medikaments zur Verwendung als 5-HT₄-Rezeptorantagonist.

15. Verwendung nach Anspruch 14 zur Verwendung als 5-HT₄-Antagonist bei der Behandlung oder Prophylaxe von Magen-Darm-Störungen, kardiovaskulären Störungen und ZNS-Störungen.

## Revendications

1. Composé de formule (I), ou un de ses sels pharmaceutiquement acceptables : 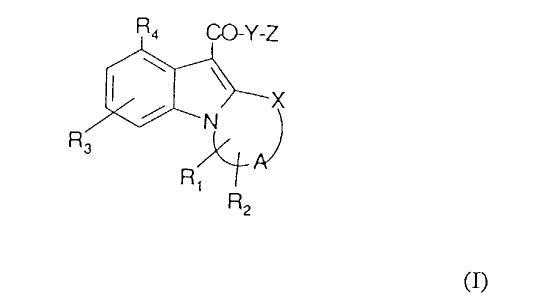 dans laquelle
X représente O, S, un groupe SO, SO₂, CH₂, CH ou NR dans lequel R représente l'hydrogène, un groupe alkyle en C₁ à C₆ ou cycloalkyle en C₃ à C₆ ;
A représente une chaîne polyméthylène saturée ou insaturée ayant 2 à 4 atomes de carbone ;
R₁ et R₂ représentent l'hydrogène, des groupes alkyle en C₁ à C₆ ou cycloalkyle en C₃ à C₆ ;
R₃ représente l'hydrogène, un groupe halogéno, alkyle en C₁ à C₆, cycloalkyle en C₃ à C₆, amino, nitro ou alkoxy en C₁ à C₆ ;
R₄ représente l'hydrogène, un groupe halogéno, alkyle en C₁ à C₆, cycloalkyle en C₃ à C₆ ou alkoxy en C₁ à C₆ ;
Y représente O ou un groupe NH ;
Z répond à la sous-formule (a), (b) ou (c) : 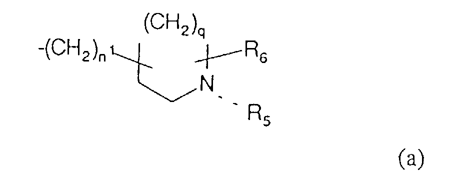 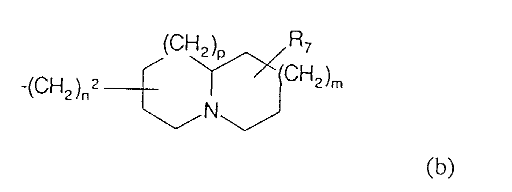 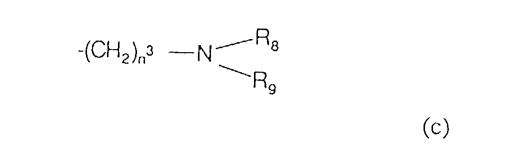
n¹ est égal à 1, 2, 3 ou 4 ; n² est égal à 0, 1, 2, 3 ou 4 ; n³ est égal à 2, 3, 4 ou 5 ;
q est égal à 0, 1, 2 ou 3 ; p est égal à 0, 1 ou 2 ; m est égal à 0, 1 ou 2 ;
R₅ représente l'hydrogène, un groupe alkyle en C₁ à C₁₂ ou aralkyle, ou bien R₅ représente un groupe (CH₂)_{z}-R₁₀ dans lequel z est égal à 2 ou 3 et R₁₀ est choisi entre des groupes cyano, hydroxyle, alkoxy en C₁ à C₆, phénoxy, C(O)alkyle en C₁ à C₆, COC₆H₅, -CONR₁₁R₁₂, NR₁₁COR₁₂, SO₂NR₁₁R₁₂ et NR₁₁SO₂R₁₂ dans lesquels R₁₁ et R₁₂ représentent l'hydrogène, des groupes alkyle en C₁ à C₆ ou cycloalkyle en C₃ à C₆ ;
R₆, R₇ et R₈ représentent, indépendamment, l'hydrogène, un groupe alkyle en C₁ à C₆ ou cycloalkyle en C₃ à C₆ ;
R₉ représente l'hydrogène, un groupe alkyle en C₁ à C₆ ou cycloalkyle en C₃ à C₆ ; et
la ligne discontinue dans la sous-formule (a) indique que le groupe R₅ est absent lorsque le groupe -(CH₂)ₙ1 est fixé directement à l'atome d'azote de l'azacycle ;
ou un composé de formule (I) dans laquelle la liaison CO-Y est remplacée par un bio-isostère hétérocyclique de formule (d) : 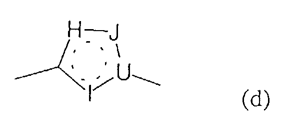 dans laquelle
le cercle en trait discontinu représente une ou deux doubles liaisons à n'importe quelle position dans le noyau pentagonal ; H, J et I représentent, indépendamment, l'oxygène, le soufre, l'azote ou le carbone, sous réserve qu'au moins un des symboles H, J et I représente un atome autre que le carbone ; U représente l'azote ou le carbone ;
autre que le composé de formule (I) dans laquelle X représente O, A représente un groupe -(CH₂)₃-, R₁, R₂, R₃ et R₄ représentent l'hydrogène, Y représente un groupe NH, Z répond à la sous-formule (a) dans laquelle n¹ est égal à 1, q est égal à 3, R₅ représente un groupe n-butyle et R₆ représente l'hydrogène ;
ayant une activité antagoniste du récepteur 5-HT₄.

2. Composé suivant la revendication 1, dans lequel X représente O.

3. Composé suivant la revendication 1 ou 2, dans lequel A représente un groupe (CH₂)₃-.

4. Composé suivant la revendication 1, 2 ou 3, dans lequel R₁ et R₂ représentent, indépendamment, l'hydrogène ou un groupe méthyle.

5. Composé suivant la revendication 1, 2, 3 ou 4, dans lequel R₃ représente l'hydrogène et R₄ représente l'hydrogène ou un groupe halogéno.

6. Composé suivant l'une quelconque des revendications 1 à 5, dans lequel Y représente O ou un groupe NH.

7. Composé suivant l'une quelconque des revendications 1 à 6, dans lequel Z répond à la sous-formule (a) et (CH₂)ₙ1 est fixé à un atome de carbone de l'azacycle.

8. Composé suivant la revendication 7, dans lequel Z représente un groupe 4-pipéridylméthyle N-substitué.

9. Composé suivant la revendication 8, dans lequel le substituant sur l'atome N est un groupe alkyle ayant deux ou plus de deux atomes de carbone ou un groupe benzyle facultativement substitué.

10. Composé suivant la revendication 1, choisi parmi les composés E1 à E46 inclus (autres que le composé E3), répondant à la définition dans le présent mémoire et dans lesquels A représente un groupe de formule -CH₂-(CH₂)ᵣ-CH₂-, y compris leurs sels pharmaceutiquement acceptables
| | **R**_{**1**} | **R**_{**2**} | **r** | **R**_{**3**} | **R**_{**4**} | **X** | **Y** | **Z** |
|---|---|---|---|---|---|---|---|---|
| **E1** | H | H | 1 | H | H | O | O | (i) |
| **E2** | H | H | 1 | H | H | O | O | (vi) |
| **E3** | H | H | 1 | H | H | O | NH | (i) |
| **E4** | H | H | 1 | H | H | O | O | (iii) |
| **E5** | H | H | 1 | H | H | O | NH | (iii) |
| **E6** | H | H | O | H | H | O | O | (i) |
| **E7** | 3-(CH₃)₂ | | 1 | H | H | O | O | (i) |
| **E8** | H | H | 1 | H | H | S | O | (i) |
| **E9** | H | H | 2 | H | H | O | O | (i) |
| **E10** | H | H | 1 | H | H | CH₂ | O | (i) |
| **E11** | H | H | O | H | H | CH₂ | O | (i) |
| **E12** | H | H | 2 | H | H | CH₂ | O | (i) |
| **E13** | H | H | O | H | H | CH₂ | NH | (i) |
| **E14** | H | H | O | H | H | O | NH | (i) |
| **E15** | H | H | 1 | H | H | O | O | Bzppm |
| **E16** | H | H | 1 | H | H | SO | O | (i) |
| **E17** | -- | Δ | - | H | H | CH | O | (i) |
| **E18** | -- | Γ | - | H | H | CH | O | (i) |
| **E19** | H | H | 1 | H | H | S | NH | (i) |
| **E20** | H | H | 1 | H | H | O | NH | Bzppm |
| **E21** | H | H | 1 | H | H | O | NH | ppm |
| **E22** | H | H | 1 | H | H | O | NH | nC₆H₁₃ppm |
| **E23** | H | H | 1 | H | H | O | NH | (ii) |
| **E24** | H | H | 1 | H | H | O | NH | Etppm |
| **E25** | H | H | 1 | H | H | O | NH | MeSO₂aEtppm |
| **E26** | H | H | 1 | H | H | O | NH | (vi) |
| **E27** | H | H | 1 | 8-F | H | O | O | (i) |
| **E28** | H | H | 1 | 8-F | H | O | NH | (i) |
| **E29** | H | H | 1 | H | H | NMe | O | (i) |
| **E30** | -- | π | -- | H | H | S | O | (i) |
| **E31** | H | H | 0 | H | H | S | O | (i) |
| **E32** | -- | ϑ | - | H | H | S | O | (i) |
| **E33** | -- | Λ | - | H | H | N | O | (i) |
| **E34** | H | H | O | H | H | S | NH | (i) |
| **E35** | -- | ϑ | -- | H | H | S | NH | (i) |
| **E36** | H | H | 1 | H | H | NH | O | (i) |
| **E37** | H | H | O | H | H | O | O | (vi) |
| **E38** | H | H | 2 | H | H | O | NH | (i) |
| **E39** | - | Γ | - | H | H | N | O | (i) |
| **E40** | H | H | O | H | H | S | O | (vi) |
| **E41** | H | H | O | H | H | S | NH | (vi) |
| **E42** | -- | ϑ | -- | H | H | S | O | (vi) |
| **E43** | -- | ϑ | -- | H | H | S | NH | (vi) |
| **E44** | H | H | 1 | H | H | S | O | (vi) |
| **E45** | -- | Γ | -- | H | H | NH | NH | (i) |
| **E46** | H | H | 1 | H | H | N | NH | (i) |
dans lesquelles les variables sont définies comme suit : 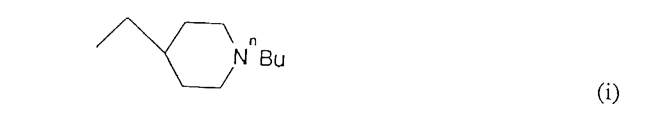 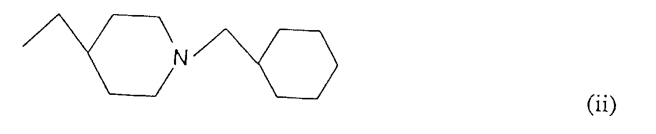 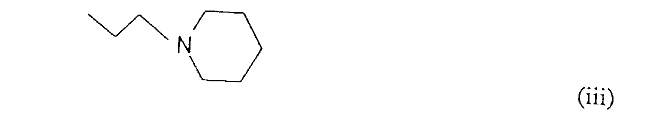 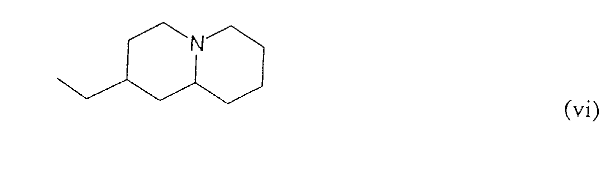 Δ - AR₁R₂ is -(CH₂)₂-CH= Bz - benzyle
Γ - AR₁R₂ is -CH=CH-CH= ppm - 4-pipéridylméthyle
π - AR₁R₂ is -C(CH₃)=CH- aEt - aminoéthyle
ϑ - AR₁R₂ is-CH=CH-
Λ - AR₁R₂ is -C(CH₃)=CH-C(CH₃)=

11. Procédé pour la préparation des esters ou amides suivant la revendication 6, qui comprend la réaction d'un dérivé d'acide indole-10-carboxylique approprié avec un alcool ou une amine approprié.

12. Composition pharmaceutique comprenant un composé suivant l'une quelconque des revendications 1 à 10 et un support pharmaceutiquement acceptable.

13. Composé suivant la revendication 1, destiné à être utilisé comme substance thérapeutique active.

14. Utilisation d'un composé suivant la revendication 1, dans la production d'un médicament destiné à être utilisé comme antagoniste des récepteurs 5-HT₄.

15. Utilisation suivant la revendication 14 comme antagoniste des récepteurs 5-HT₄ dans le traitement ou la prophylaxie de troubles gastro-intestinaux, de troubles cardiovasculaires et de troubles du système nerveux central (SNC).
